(19) Europäisches Patentamt
European Patent Office
Office européen des brevets

(11) EP 2 243 792 A1

(12) EUROPEAN PATENT APPLICATION

(43) Date of publication:
**27.10.2010 Bulletin 2010/43**

(51) Int Cl.:
***C07K 14/435** (2006.01)* ***D01F 4/00** (2006.01)*
***C12N 15/62** (2006.01)*

(21) Application number: **09158445.8**

(22) Date of filing: **22.04.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(71) Applicant: **Spiber Technologies AB**
**756 51 Uppsala (SE)**

(72) Inventors:
- **Johansson, Jan**
**116 31 Stockholm (SE)**
- **Rising, Anna**
**756 51 Uppsala (SE)**
- **Hedhammar, My**
**113 51 Stockholm (SE)**

(74) Representative: **Henriksson, Mikael**
**AWAPATENT AB**
**P.O. Box 45806**
**104 30 Stockholm (SE)**

(54) **Methods of producing polymers of spider silk proteins**

(57) A method of producing polymers of an isolated spider silk protein consisting of from 170 to 600 amino acid residues involves providing a solution of said spider silk protein in a liquid medium at pH 6.4 or higher and/or an ion composition that prevents polymerisation of the spider silk protein. The properties of the liquid medium are adjusted to a pH of 6.3 or lower and an ion composition that allows polymerisation of the spider silk protein. The spider silk protein is allowed to form solid polymers in the liquid medium, and the resulting solid spider silk protein polymers are isolated from the liquid medium. The resulting polymers are useful as fibers, films, foams, nets or meshes.

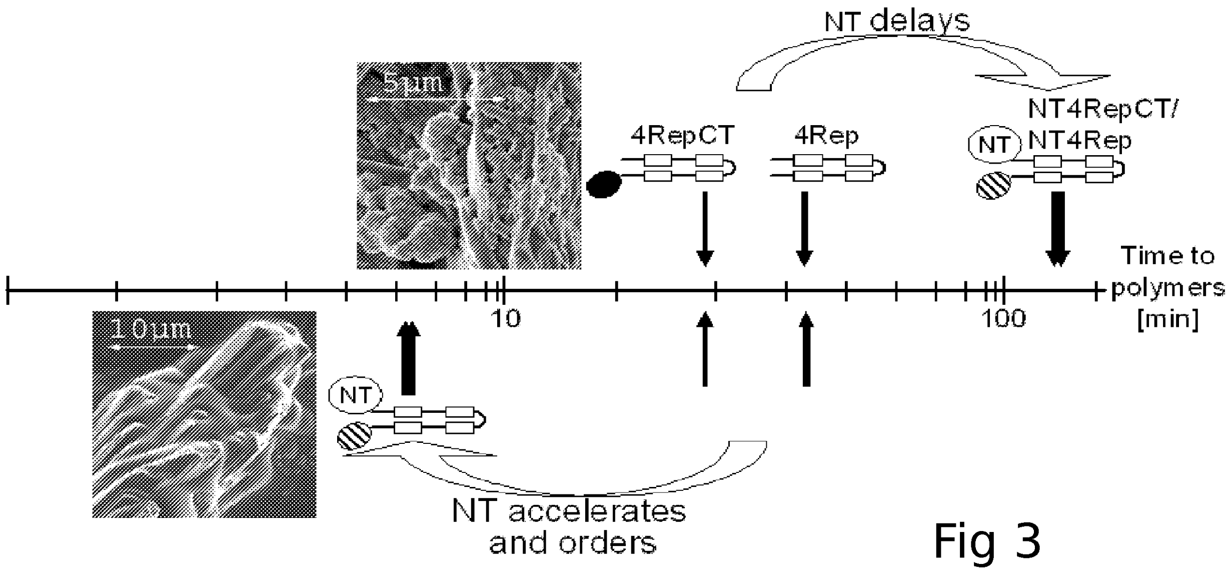


Fig 3

EP 2 243 792 A1

SEQUENCE LISTING

```
<110>  Spiber Technologies AB

<120>  METHOD OF PRODUCING POLYMERS OF SPIDER SILK PROTEINS

<130>  EP-21041052

<160>  16

<170>  PatentIn version 3.5

<210>  1
<211>  149
<212>  PRT
<213>  Euprosthenops australis

<400>  1

Gly Ser Gly Asn Ser Gly Ile Gln Gly Gln Gly Gly Tyr Gly Gly Leu
1               5                   10                  15

Gly Gln Gly Gly Tyr Gly Gln Gly Ala Gly Ser Ser Ala Ala Ala Ala
            20                  25                  30

Ala Ala Ala Ala Ala Ala Ala Ala Gly Gly Gln Gly Gly Gln Gly Gln
        35                  40                  45

Gly Gly Tyr Gly Gln Gly Ser Gly Gly Ser Ala Ala Ala Ala Ala Ala
    50                  55                  60

Ala Ala Ala Ala Ala Ala Ala Ala Ala Gly Arg Gly Gln Gly Gly Tyr
65                  70                  75                  80

Gly Gln Gly Ser Gly Gly Asn Ala Ala Ala Ala Ala Ala Ala Ala Ala
                85                  90                  95

Ala Ala Ala Ala Ala Gly Gln Gly Gly Gln Gly Gly Tyr Gly Arg Gln
            100                 105                 110

Ser Gln Gly Ala Gly Ser Ala Ala Ala Ala Ala Ala Ala Ala Ala Ala
        115                 120                 125

Ala Ala Ala Ala Gly Ser Gly Gln Gly Gly Tyr Gly Gln Gly Gln Gly
    130                 135                 140

Gly Tyr Gly Gln Ser
145


<210>  2
<211>  265
<212>  PRT
<213>  Euprosthenops australis


<220>
<221>  DOMAIN
<222>  (1)..(167)
<223>  REP fragment
```

<220>
<221> DOMAIN
<222> (168)..(265)
<223> CT fragment

<400> 2

```
Gly Ser Gly Asn Ser Gly Ile Gln Gly Gln Gly Gly Tyr Gly Gly Leu
1               5                   10                  15

Gly Gln Gly Gly Tyr Gly Gln Gly Ala Gly Ser Ser Ala Ala Ala Ala
            20                  25                  30

Ala Ala Ala Ala Ala Ala Ala Ala Gly Gly Gln Gly Gly Gln Gly Gln
        35                  40                  45

Gly Gly Tyr Gly Gln Gly Ser Gly Gly Ser Ala Ala Ala Ala Ala Ala
    50                  55                  60

Ala Ala Ala Ala Ala Ala Ala Ala Ala Gly Arg Gly Gln Gly Gly Tyr
65                  70                  75                  80

Gly Gln Gly Ser Gly Gly Asn Ala Ala Ala Ala Ala Ala Ala Ala Ala
                85                  90                  95

Ala Ala Ala Ala Ala Gly Gln Gly Gly Gln Gly Gly Tyr Gly Arg Gln
            100                 105                 110

Ser Gln Gly Ala Gly Ser Ala Ala Ala Ala Ala Ala Ala Ala Ala Ala
        115                 120                 125

Ala Ala Ala Ala Gly Ser Gly Gln Gly Gly Tyr Gly Gln Gly Gln Gly
    130                 135                 140

Gly Tyr Gly Gln Ser Ser Ala Ser Ala Ser Ala Ala Ala Ser Ala Ala
145                 150                 155                 160

Ser Thr Val Ala Asn Ser Val Ser Arg Leu Ser Ser Pro Ser Ala Val
                165                 170                 175

Ser Arg Val Ser Ser Ala Val Ser Ser Leu Val Ser Asn Gly Gln Val
            180                 185                 190

Asn Met Ala Ala Leu Pro Asn Ile Ile Ser Asn Ile Ser Ser Ser Val
        195                 200                 205

Ser Ala Ser Ala Pro Gly Ala Ser Gly Cys Glu Val Ile Val Gln Ala
    210                 215                 220

Leu Leu Glu Val Ile Thr Ala Leu Val Gln Ile Val Ser Ser Ser Ser
225                 230                 235                 240

Val Gly Tyr Ile Asn Pro Ser Ala Val Asn Gln Ile Thr Asn Val Val
                245                 250                 255
```

```
Ala Asn Ala Met Ala Gln Val Met Gly
            260                 265


<210>  3
<211>  296
<212>  PRT
<213>  Euprosthenops australis


<220>
<221>  DOMAIN
<222>  (1)..(137)
<223>  NT fragment

<220>
<221>  DOMAIN
<222>  (138)..(296)
<223>  REP fragment

<400>  3

Gly Ser Gly Asn Ser His Thr Thr Pro Trp Thr Asn Pro Gly Leu Ala
1               5                   10                  15


Glu Asn Phe Met Asn Ser Phe Met Gln Gly Leu Ser Ser Met Pro Gly
            20                  25                  30


Phe Thr Ala Ser Gln Leu Asp Asp Met Ser Thr Ile Ala Gln Ser Met
        35                  40                  45


Val Gln Ser Ile Gln Ser Leu Ala Ala Gln Gly Arg Thr Ser Pro Asn
    50                  55                  60


Lys Leu Gln Ala Leu Asn Met Ala Phe Ala Ser Ser Met Ala Glu Ile
65                  70                  75                  80


Ala Ala Ser Glu Glu Gly Gly Gly Ser Leu Ser Thr Lys Thr Ser Ser
                85                  90                  95


Ile Ala Ser Ala Met Ser Asn Ala Phe Leu Gln Thr Thr Gly Val Val
            100                 105                 110


Asn Gln Pro Phe Ile Asn Glu Ile Thr Gln Leu Val Ser Met Phe Ala
        115                 120                 125


Gln Ala Gly Met Asn Asp Val Ser Ala Ser Ala Ser Ala Gly Ala Ser
    130                 135                 140


Ala Ala Ala Ser Ala Gly Ala Ala Ser Gly Gln Gly Gly Tyr Gly Gly
145                 150                 155                 160


Leu Gly Gln Gly Gly Tyr Gly Gln Gly Ala Gly Ser Ser Ala Ala Ala
                165                 170                 175


Ala Ala Ala Ala Ala Ala Ala Ala Ala Gly Gly Gln Gly Gly Gln Gly
            180                 185                 190
```

Gln Gly Gly Tyr Gly Gln Gly Ser Gly Gly Ser Ala Ala Ala Ala Ala
195 200 205

Ala Ala Ala Ala Ala Ala Ala Ala Ala Ala Gly Arg Gly Gln Gly Gly
210 215 220

Tyr Gly Gln Gly Ser Gly Gly Asn Ala Ala Ala Ala Ala Ala Ala Ala
225 230 235 240

Ala Ala Ala Ala Ala Ala Ala Gly Gln Gly Gly Gln Gly Gly Tyr Gly
245 250 255

Arg Gln Ser Gln Gly Ala Gly Ser Ala Ala Ala Ala Ala Ala Ala Ala
260 265 270

Ala Ala Ala Ala Ala Ala Gly Ser Gly Gln Gly Gly Tyr Gly Gly Gln
275 280 285

Gly Gln Gly Gly Tyr Gly Gln Ser
290 295

<210> 4
<211> 340
<212> PRT
<213> Euprosthenops australis

<220>
<221> DOMAIN
<222> (1)..(137)
<223> NT fragment

<220>
<221> DOMAIN
<222> (138)..(340)
<223> REP fragment

<400> 4

Gly Ser Gly Asn Ser His Thr Thr Pro Trp Thr Asn Pro Gly Leu Ala
1 5 10 15

Glu Asn Phe Met Asn Ser Phe Met Gln Gly Leu Ser Ser Met Pro Gly
20 25 30

Phe Thr Ala Ser Gln Leu Asp Asp Met Ser Thr Ile Ala Gln Ser Met
35 40 45

Val Gln Ser Ile Gln Ser Leu Ala Ala Gln Gly Arg Thr Ser Pro Asn
50 55 60

Lys Leu Gln Ala Leu Asn Met Ala Phe Ala Ser Ser Met Ala Glu Ile
65 70 75 80

Ala Ala Ser Glu Glu Gly Gly Gly Ser Leu Ser Thr Lys Thr Ser Ser
85 90 95

```
Ile Ala Ser Ala Met Ser Asn Ala Phe Leu Gln Thr Thr Gly Val Val
            100                 105                 110

Asn Gln Pro Phe Ile Asn Glu Ile Thr Gln Leu Val Ser Met Phe Ala
        115                 120                 125

Gln Ala Gly Met Asn Asp Val Ser Ala Ser Ala Ser Ala Gly Ala Ser
    130                 135                 140

Ala Ala Ala Ser Ala Gly Ala Pro Gly Tyr Ser Pro Ala Pro Ser Tyr
145                 150                 155                 160

Ser Ser Gly Gly Tyr Ala Ser Ser Ala Ala Ser Ala Ala Ala Ala Ala
                165                 170                 175

Gly Gln Gly Gly Pro Gly Gly Tyr Gly Pro Ala Pro Asn Gln Gly Ala
            180                 185                 190

Ser Ser Ala Ala Ala Ala Ala Ala Gly Ser Gly Gln Gly Pro Ser Gly
        195                 200                 205

Pro Tyr Gly Thr Ser Tyr Gln Ile Ser Thr Gln Tyr Thr Gln Thr Thr
    210                 215                 220

Thr Ser Gln Gly Gln Gly Tyr Gly Ser Ser Ser Ala Gly Ala Ala Ala
225                 230                 235                 240

Ala Gly Ala Ala Gly Ala Gly Gln Gly Gly Tyr Gly Gly Gln Gly Gln
                245                 250                 255

Gly Gly Tyr Gly Gln Gly Ala Gly Gly Ala Ala Ala Ala Ala Ala Ala
            260                 265                 270

Ala Ala Ala Ala Ala Ala Ala Ala Gly Gln Gly Gly Gln Gly Gly Gly
        275                 280                 285

Gly Tyr Gly Gln Gly Gly Gln Gly Gly Gln Gly Gly Gln Gly Gln Gly
    290                 295                 300

Gly Tyr Gly Gln Gly Ala Gly Ser Ser Ala Ala Ala Ala Ala Ala Ala
305                 310                 315                 320

Ala Ala Ala Ala Ala Ala Ala Gly Arg Gly Gln Gly Gly Tyr Gly Pro
                325                 330                 335

Gly Ser Gly Gly
            340
```

<210> 5
<211> 424
<212> PRT
<213> Euprosthenops australis

<220>
<221> DOMAIN
<222> (1)..(136)
<223> NT fragment

<220>
<221> DOMAIN
<222> (137)..(313)
<223> REP fragment

<220>
<221> DOMAIN
<222> (314)..(411)
<223> CT fragment

<220>
<221> DOMAIN
<222> (412)..(424)
<223> His tag

<400> 5

```
Met Lys Ala Ser His Thr Thr Pro Trp Thr Asn Pro Gly Leu Ala Glu
1               5                   10                  15

Asn Phe Met Asn Ser Phe Met Gln Gly Leu Ser Ser Met Pro Gly Phe
            20                  25                  30

Thr Ala Ser Gln Leu Asp Asp Met Ser Thr Ile Ala Gln Ser Met Val
        35                  40                  45

Gln Ser Ile Gln Ser Leu Ala Ala Gln Gly Arg Thr Ser Pro Asn Lys
    50                  55                  60

Leu Gln Ala Leu Asn Met Ala Phe Ala Ser Ser Met Ala Glu Ile Ala
65                  70                  75                  80

Ala Ser Glu Glu Gly Gly Gly Ser Leu Ser Thr Lys Thr Ser Ser Ile
                85                  90                  95

Ala Ser Ala Met Ser Asn Ala Phe Leu Gln Thr Thr Gly Val Val Asn
            100                 105                 110

Gln Pro Phe Ile Asn Glu Ile Thr Gln Leu Val Ser Met Phe Ala Gln
        115                 120                 125

Ala Gly Met Asn Asp Val Ser Ala Ser Ala Ser Ala Gly Ala Ser Ala
    130                 135                 140

Ala Ala Ser Ala Gly Ala Ala Ser Gly Gln Gly Gly Tyr Gly Gly Leu
145                 150                 155                 160

Gly Gln Gly Gly Tyr Gly Gln Gly Ala Gly Ser Ser Ala Ala Ala Ala
                165                 170                 175

Ala Ala Ala Ala Ala Ala Ala Ala Gly Gly Gln Gly Gly Gln Gly Gln
            180                 185                 190
```

Gly Gly Tyr Gly Gln Gly Ser Gly Gly Ser Ala Ala Ala Ala Ala Ala
195 200 205

Ala Ala Ala Ala Ala Ala Ala Ala Ala Gly Arg Gly Gln Gly Gly Tyr
210 215 220

Gly Gln Gly Ser Gly Gly Asn Ala Ala Ala Ala Ala Ala Ala Ala Ala
225 230 235 240

Ala Ala Ala Ala Ala Ala Gly Gln Gly Gly Gln Gly Gly Tyr Gly Arg
245 250 255

Gln Ser Gln Gly Ala Gly Ser Ala Ala Ala Ala Ala Ala Ala Ala Ala
260 265 270

Ala Ala Ala Ala Ala Gly Ser Gly Gln Gly Gly Tyr Gly Gly Gln Gly
275 280 285

Gln Gly Gly Tyr Gly Gln Ser Ser Ala Ser Ala Ser Ala Ala Ala Ser
290 295 300

Ala Ala Ser Thr Val Ala Asn Ser Val Ser Arg Leu Ser Ser Pro Ser
305 310 315 320

Ala Val Ser Arg Val Ser Ser Ala Val Ser Ser Leu Val Ser Asn Gly
325 330 335

Gln Val Asn Met Ala Ala Leu Pro Asn Ile Ile Ser Asn Ile Ser Ser
340 345 350

Ser Val Ser Ala Ser Ala Pro Gly Ala Ser Gly Cys Glu Val Ile Val
355 360 365

Gln Ala Leu Leu Glu Val Ile Thr Ala Leu Val Gln Ile Val Ser Ser
370 375 380

Ser Ser Val Gly Tyr Ile Asn Pro Ser Ala Val Asn Gln Ile Thr Asn
385 390 395 400

Val Val Ala Asn Ala Met Ala Gln Val Met Gly Lys Leu Ala Ala Ala
405 410 415

Leu Glu His His His His His His
420

<210> 6
<211> 137
<212> PRT
<213> Euprosthenops australis

<220>
<221> VARIANT
<222> (6)..(6)

<223> deletion (deltaHis)

<400> 6

Gly Ser Gly Asn Ser His Thr Thr Pro Trp Thr Asn Pro Gly Leu Ala
1 5 10 15

Glu Asn Phe Met Asn Ser Phe Met Gln Gly Leu Ser Ser Met Pro Gly
20 25 30

Phe Thr Ala Ser Gln Leu Asp Asp Met Ser Thr Ile Ala Gln Ser Met
35 40 45

Val Gln Ser Ile Gln Ser Leu Ala Ala Gln Gly Arg Thr Ser Pro Asn
50 55 60

Lys Leu Gln Ala Leu Asn Met Ala Phe Ala Ser Ser Met Ala Glu Ile
65 70 75 80

Ala Ala Ser Glu Glu Gly Gly Gly Ser Leu Ser Thr Lys Thr Ser Ser
85 90 95

Ile Ala Ser Ala Met Ser Asn Ala Phe Leu Gln Thr Thr Gly Val Val
100 105 110

Asn Gln Pro Phe Ile Asn Glu Ile Thr Gln Leu Val Ser Met Phe Ala
115 120 125

Gln Ala Gly Met Asn Asp Val Ser Ala
130 135

<210> 7
<211> 98
<212> PRT
<213> Euprosthenops australis

<400> 7

Ser Arg Leu Ser Ser Pro Ser Ala Val Ser Arg Val Ser Ser Ala Val
1 5 10 15

Ser Ser Leu Val Ser Asn Gly Gln Val Asn Met Ala Ala Leu Pro Asn
20 25 30

Ile Ile Ser Asn Ile Ser Ser Ser Val Ser Ala Ser Ala Pro Gly Ala
35 40 45

Ser Gly Cys Glu Val Ile Val Gln Ala Leu Leu Glu Val Ile Thr Ala
50 55 60

Leu Val Gln Ile Val Ser Ser Ser Ser Val Gly Tyr Ile Asn Pro Ser
65 70 75 80

Ala Val Asn Gln Ile Thr Asn Val Val Ala Asn Ala Met Ala Gln Val
85 90 95

Met Gly

<210> 8
<211> 131
<212> PRT
<213> Artificial Sequence

<220>
<223> Consensus sequence derived from spidroin NT fragments

<220>
<221> VARIANT
<222> (20)..(20)
<223> Leu

<220>
<221> VARIANT
<222> (42)..(42)
<223> Asn

<220>
<221> VARIANT
<222> (42)..(42)
<223> Gln

<220>
<221> VARIANT
<222> (50)..(50)
<223> Ser

<220>
<221> VARIANT
<222> (50)..(50)
<223> Lys

<220>
<221> VARIANT
<222> (56)..(56)
<223> Arg

<220>
<221> VARIANT
<222> (84)..(84)
<223> Leu

<220>
<221> VARIANT
<222> (114)..(114)
<223> Ser

<220>
<221> VARIANT
<222> (121)..(121)
<223> Asn

<220>
<221> VARIANT
<222> (123)..(123)
<223> Leu

<220>
<221> VARIANT
<222> (124)..(124)
<223> Ser

<400> 8

Gln Ala Asn Thr Pro Trp Ser Ser Pro Asn Leu Ala Asp Ala Phe Ile
1               5                   10                  15

Asn Ser Phe Met Ser Ala Ala Ser Ser Ser Gly Ala Phe Ser Ala Asp
            20                  25                  30

Gln Leu Asp Asp Met Ser Thr Ile Gly Asp Thr Leu Met Ser Ala Met
        35                  40                  45

Asp Asn Met Gly Arg Ser Gly Lys Ser Thr Lys Ser Lys Leu Gln Ala
    50                  55                  60

Leu Asn Met Ala Phe Ala Ser Ser Met Ala Glu Ile Ala Ala Ala Glu
65                  70                  75                  80

Ser Gly Gly Gly Ser Val Gly Val Lys Thr Asn Ala Ile Ser Asp Ala
                85                  90                  95

Leu Ser Ser Ala Phe Tyr Gln Thr Thr Gly Ser Val Asn Pro Gln Phe
            100                 105                 110

Val Asn Glu Ile Arg Ser Leu Ile Gly Met Phe Ala Gln Ala Ser Ala
        115                 120                 125

Asn Glu Val
    130

<210> 9
<211> 100
<212> PRT
<213> Artificial Sequence

<220>
<223> Consensus sequence derived from known MaSp1 and MaSp2 proteins

<220>
<221> MISC_FEATURE
<222> (1)..(71)
<223> Sequence length present in known species variants

<220>
<221> VARIANT
<222> (7)..(7)
<223> Glu

<400> 9

Ser Arg Leu Ser Ser Pro Gln Ala Ser Ser Arg Val Ser Ser Ala Val
1               5                   10                  15

Ser Asn Leu Val Ser Ser Gly Pro Thr Asn Ser Ala Ala Leu Ser Asn
            20                  25                  30

Thr Ile Ser Asn Val Val Ser Gln Ile Ser Ala Ser Asn Pro Gly Leu
        35                  40                  45

```
Ser Gly Cys Asp Val Leu Val Gln Ala Leu Leu Glu Val Val Ser Ala
    50                  55                  60

Leu Val His Ile Leu Gly Ser Ser Ser Ile Gly Gln Val Asn Tyr Gly
65                  70                  75                  80

Ser Ala Gly Gln Ala Thr Gln Ile Val Gly Gln Ser Val Ala Gln Ala
                85                  90                  95

Leu Gly Glu Phe
            100
```

<210> 10
<211> 1110
<212> PRT
<213> Euprosthenops australis

<220>
<221> REPEAT
<222> (7)..(19)

<220>
<221> REPEAT
<222> (20)..(42)

<220>
<221> REPEAT
<222> (43)..(56)

<220>
<221> REPEAT
<222> (57)..(70)

<220>
<221> REPEAT
<222> (71)..(83)

<220>
<221> REPEAT
<222> (84)..(106)

<220>
<221> REPEAT
<222> (107)..(120)

<220>
<221> REPEAT
<222> (121)..(134)

<220>
<221> REPEAT
<222> (135)..(147)

<220>
<221> REPEAT
<222> (148)..(170)

<220>
<221> REPEAT
<222> (171)..(183)

<220>
<221> REPEAT
<222> (184)..(197)

<220>
<221> REPEAT
<222> (198)..(211)

<220>
<221> REPEAT
<222> (212)..(234)

<220>
<221> REPEAT
<222> (235)..(248)

<220>
<221> REPEAT
<222> (249)..(265)

<220>
<221> REPEAT
<222> (266)..(279)

<220>
<221> REPEAT
<222> (280)..(293)

<220>
<221> REPEAT
<222> (294)..(306)

<220>
<221> REPEAT
<222> (307)..(329)

<220>
<221> REPEAT
<222> (330)..(342)

<220>
<221> REPEAT
<222> (343)..(356)

<220>
<221> REPEAT
<222> (357)..(370)

<220>
<221> REPEAT
<222> (371)..(393)

<220>
<221> REPEAT
<222> (394)..(406)

<220>
<221> REPEAT
<222> (407)..(420)

<220>
<221> REPEAT
<222> (421)..(434)

<220>
<221> REPEAT
<222> (435)..(457)

<220>
<221> REPEAT
<222> (458)..(470)

<220>
<221> REPEAT

<222> (471)..(488)

<220>
<221> REPEAT
<222> (489)..(502)

<220>
<221> REPEAT
<222> (503)..(516)

<220>
<221> REPEAT
<222> (517)..(529)

<220>
<221> REPEAT
<222> (530)..(552)

<220>
<221> REPEAT
<222> (553)..(566)

<220>
<221> REPEAT
<222> (567)..(580)

<220>
<221> REPEAT
<222> (581)..(594)

<220>
<221> REPEAT
<222> (595)..(617)

<220>
<221> REPEAT
<222> (618)..(630)

<220>
<221> REPEAT
<222> (631)..(647)

<220>
<221> REPEAT
<222> (648)..(661)

<220>
<221> REPEAT
<222> (662)..(675)

<220>
<221> REPEAT
<222> (676)..(688)

<220>
<221> REPEAT
<222> (689)..(711)

<220>
<221> REPEAT
<222> (712)..(725)

<220>
<221> REPEAT
<222> (726)..(739)

<220>
<221> REPEAT
<222> (740)..(752)

<220>
<221> REPEAT
<222> (753)..(775)

<220>
<221> REPEAT
<222> (776)..(789)

<220>
<221> REPEAT
<222> (790)..(803)

<220>
<221> REPEAT
<222> (804)..(816)

<220>
<221> REPEAT
<222> (817)..(839)

<220>
<221> REPEAT
<222> (840)..(853)

<220>
<221> REPEAT
<222> (854)..(867)

<220>
<221> REPEAT
<222> (868)..(880)

<220>
<221> REPEAT
<222> (881)..(903)

<220>
<221> REPEAT
<222> (904)..(917)

<220>
<221> REPEAT
<222> (918)..(931)

<220>
<221> REPEAT
<222> (932)..(945)

<220>
<221> REPEAT
<222> (946)..(968)

<220>
<221> REPEAT
<222> (969)..(981)

<220>
<221> REPEAT
<222> (982)..(998)

<220>
<221> REPEAT
<222> (999)..(1013)

<220>
<221> REPEAT
<222> (1014)..(1027)

<220>
<221> REPEAT

<222> (1028)..(1042)

<220>
<221> REPEAT
<222> (1043)..(1059)

<220>
<221> REPEAT
<222> (1060)..(1073)

<220>
<221> REPEAT
<222> (1074)..(1092)

<400> 10

```
Gln Gly Ala Gly Gly Asn Ala Ala Ala Ala Ala Ala Ala Ala Ala Ala
1               5                   10                  15

Ala Ala Ala Gly Gln Gly Gly Gln Gly Gly Tyr Gly Gly Leu Gly Gln
            20                  25                  30

Gly Gly Tyr Gly Gln Gly Ala Gly Ser Ser Ala Ala Ala Ala Ala Ala
        35                  40                  45

Ala Ala Ala Ala Ala Ala Ala Ala Gly Arg Gly Gln Gly Gly Tyr Gly
    50                  55                  60

Gln Gly Ser Gly Gly Asn Ala Ala Ala Ala Ala Ala Ala Ala Ala Ala
65                  70                  75                  80

Ala Ala Ser Gly Gln Gly Gly Gln Gly Gly Gln Gly Gly Gln Gly Gln
                85                  90                  95

Gly Gly Tyr Gly Gln Gly Ala Gly Ser Ser Ala Ala Ala Ala Ala Ala
            100                 105                 110

Ala Ala Ala Ala Ala Ala Ala Ala Gly Gln Gly Gln Gly Arg Tyr Gly
        115                 120                 125

Gln Gly Ala Gly Gly Asn Ala Ala Ala Ala Ala Ala Ala Ala Ala Ala
    130                 135                 140

Ala Ala Ala Gly Gln Gly Gly Gln Gly Gly Gln Gly Gly Leu Gly Gln
145                 150                 155                 160

Gly Gly Tyr Gly Gln Gly Ala Gly Ser Ser Ala Ala Ala Ala Ala Ala
                165                 170                 175

Ser Ala Ala Ala Ala Ala Ala Gly Arg Gly Gln Gly Gly Tyr Gly Gln
            180                 185                 190

Gly Ala Gly Gly Asn Ala Ala Ala Ala Ala Ala Ala Ala Ala Ala Ala
        195                 200                 205

Ala Ala Ala Gly Gln Gly Gly Gln Gly Gly Tyr Gly Gly Leu Gly Gln
    210                 215                 220
```

```
Gly Gly Tyr Gly Gln Gly Ala Gly Ser Ser Ala Ala Ala Ala Ala Ala
225                 230                 235                 240

Ala Ala Ala Ala Ala Ala Ala Gly Gly Gln Gly Gly Gln Gly Gln Gly
                245                 250                 255

Arg Tyr Gly Gln Gly Ala Gly Ser Ser Ala Ala Ala Ala Ala Ala Ala
            260                 265                 270

Ala Ala Ala Ala Ala Ala Ala Gly Gln Gly Gln Gly Gly Tyr Gly Gln
        275                 280                 285

Gly Ala Gly Gly Asn Ala Ala Ala Ala Ala Ala Ala Ala Ala Ala Ala
    290                 295                 300

Ala Ala Gly Gln Gly Gly Gln Gly Gly Gln Gly Gly Leu Gly Gln Gly
305                 310                 315                 320

Gly Tyr Gly Gln Gly Ala Gly Ser Ser Ala Ala Ala Ala Ala Ala Ala
                325                 330                 335

Ala Ala Ala Ala Ala Ala Gly Arg Gly Gln Gly Gly Tyr Gly Gln Gly
            340                 345                 350

Ala Gly Gly Asn Ala Ala Ala Ala Ala Ala Ala Ala Ala Glu Ala Ala
        355                 360                 365

Ala Ala Gly Gln Gly Gly Gln Gly Gly Tyr Gly Gly Leu Gly Gln Gly
    370                 375                 380

Gly Tyr Gly Gln Gly Ala Gly Ser Ser Ala Ala Ala Ala Ala Ala Ala
385                 390                 395                 400

Ala Ala Ala Ala Ala Ala Gly Arg Gly Gln Gly Gly Tyr Gly Gln Gly
                405                 410                 415

Ala Gly Gly Asn Ala Ala Ala Ala Ala Ala Ala Ala Ala Ala Ala Ala
            420                 425                 430

Ala Ala Gly Gln Gly Gly Gln Gly Gly Tyr Gly Gly Leu Gly Gln Gly
        435                 440                 445

Gly Tyr Gly Gln Gly Ala Gly Ser Ser Ala Ala Ala Ala Ala Ala Ala
    450                 455                 460

Ala Ala Ala Ala Ala Ala Gly Gly Gln Gly Gly Gln Gly Gln Gly Arg
465                 470                 475                 480

Tyr Gly Gln Gly Ala Gly Ser Ser Ala Ala Ala Ala Ala Ala Ala Ala
                485                 490                 495
```

Ala Ala Ala Ala Ala Ala Gly Arg Gly Gln Gly Gly Tyr Gly Gln Gly
500 505 510

Ser Gly Gly Asn Ala Ala Ala Ala Ala Ala Ala Ala Ala Ala Ala Ala
515 520 525

Ser Gly Gln Gly Ser Gln Gly Gly Gln Gly Gly Gln Gly Gln Gly Gly
530 535 540

Tyr Gly Gln Gly Ala Gly Ser Ser Ala Ala Ala Ala Ala Ala Ala Ala
545 550 555 560

Ala Ala Ala Ala Ala Ser Gly Arg Gly Gln Gly Gly Tyr Gly Gln Gly
565 570 575

Ala Gly Gly Asn Ala Ala Ala Ala Ala Ala Ala Ala Ala Ala Ala Ala
580 585 590

Ala Ala Gly Gln Gly Gly Gln Gly Gly Tyr Gly Gly Leu Gly Gln Gly
595 600 605

Gly Tyr Gly Gln Gly Ala Gly Ser Ser Ala Ala Ala Ala Ala Ala Ala
610 615 620

Ala Ala Ala Ala Ala Gly Gly Gln Gly Gly Gln Gly Gln Gly Gly Tyr
625 630 635 640

Gly Gln Gly Ala Gly Ser Ser Ala Ala Ala Ala Ala Ala Ala Ala Ala
645 650 655

Ala Ala Ala Ala Ala Gly Arg Gly Gln Gly Gly Tyr Gly Gln Gly Ser
660 665 670

Gly Gly Asn Ala Ala Ala Ala Ala Ala Ala Ala Ala Ala Ala Ala Ser
675 680 685

Gly Gln Gly Gly Gln Gly Gly Gln Gly Gly Gln Gly Gln Gly Gly Tyr
690 695 700

Gly Gln Gly Ala Gly Ser Ser Ala Ala Ala Ala Ala Ala Ala Ala Ala
705 710 715 720

Ala Ala Ala Ala Ala Gly Gln Gly Gln Gly Gly Tyr Gly Gln Gly Ala
725 730 735

Gly Gly Asn Ala Ala Ala Ala Ala Ala Ala Ala Ala Ala Ala Ala Ala
740 745 750

Gly Gln Gly Gly Gln Gly Gly Gln Gly Gly Leu Gly Gln Gly Gly Tyr
755 760 765

Gly Gln Gly Ala Gly Ser Ser Ala Ala Ala Ala Ala Ala Ala Ala Ala
770 775 780

```
Ala Ala Ala Ala Ala Gly Arg Gly Gln Gly Gly Tyr Gly Gln Gly Val
785                 790                 795                 800

Gly Gly Asn Ala Ala Ala Ala Ala Ala Ala Ala Ala Ala Ala Ala Ala
                805                 810                 815

Gly Gln Gly Gly Gln Gly Gly Gln Gly Gly Leu Gly Gln Gly Gly Tyr
            820                 825                 830

Gly Gln Gly Ala Gly Ser Ser Ala Ala Ala Ala Ala Ala Ala Ala Ala
        835                 840                 845

Ala Ala Ala Ala Ala Gly Arg Gly Gln Gly Gly Tyr Gly Gln Gly Ser
    850                 855                 860

Gly Gly Asn Ala Ala Ala Ala Ala Ala Ala Ala Ala Ala Ala Ala Ser
865                 870                 875                 880

Gly Gln Gly Ser Gln Gly Gly Gln Gly Gly Gln Gly Gln Gly Gly Tyr
                885                 890                 895

Gly Gln Gly Ala Gly Ser Ser Ala Ala Ala Ala Ala Ala Ala Ala Ala
            900                 905                 910

Ala Ala Ala Ala Ser Gly Arg Gly Gln Gly Gly Tyr Gly Gln Gly Ala
        915                 920                 925

Gly Gly Asn Ala Ala Ala Ala Ala Ala Ala Ala Ala Ala Ala Ala Ala
    930                 935                 940

Ala Gly Gln Gly Gly Gln Gly Gly Tyr Gly Gly Leu Gly Gln Gly Gly
945                 950                 955                 960

Tyr Gly Gln Gly Ala Gly Ser Ser Ala Ala Ala Ala Ala Ala Ala Ala
                965                 970                 975

Ala Ala Ala Ala Gly Gly Gln Gly Gly Gln Gly Gln Gly Gly Tyr Gly
            980                 985                 990

Gln Gly Ser Gly Gly Ser Ala Ala  Ala Ala Ala Ala Ala  Ala Ala Ala
        995                 1000                 1005

Ala Ala  Ala Ala Ala Gly Arg  Gly Gln Gly Gly Tyr  Gly Gln Gly
    1010                 1015                 1020

Ser Gly  Gly Asn Ala Ala Ala  Ala Ala Ala Ala Ala  Ala Ala Ala
    1025                 1030                 1035

Ala Ala  Ala Ala Gly Gln Gly  Gly Gln Gly Gly Tyr  Gly Arg Gln
    1040                 1045                 1050
```

```
Ser Gln  Gly Ala Gly Ser Ala  Ala Ala Ala Ala Ala  Ala Ala Ala
    1055                 1060                 1065

Ala Ala  Ala Ala Ala Gly Ser  Gly Gln Gly Gly Tyr  Gly Gly Gln
    1070                 1075                 1080

Gly Gln  Gly Gly Tyr Gly Gln  Ser Ser Ala Ser Ala  Ser Ala Ala
    1085                 1090                 1095

Ala Ser  Ala Ala Ser Thr Val  Ala Asn Ser Val Ser
    1100                 1105                 1110
```

<210> 11
<211> 23
<212> PRT
<213> Artificial Sequence

<220>
<223> Consensus sequence derived from internal repeats of Euprosthenops australis MaSp1

<220>
<221> VARIANT
<222> (4)..(4)
<223> Ser

<220>
<221> VARIANT
<222> (8)..(8)
<223> Tyr

<220>
<221> VARIANT
<222> (11)..(11)
<223> Gln

<400> 11

```
Gly Gln Gly Gly Gln Gly Gly Gln Gly Gly Leu Gly Gln Gly Gly Tyr
1               5                   10                  15

Gly Gln Gly Ala Gly Ser Ser
            20
```

<210> 12
<211> 17
<212> PRT
<213> Artificial Sequence

<220>
<223> Consensus sequence derived from internal repeats of Euprosthenops australis MaSp1

<220>
<221> VARIANT
<222> (9)..(9)
<223> Arg

<220>
<221> VARIANT
<222> (14)..(14)
<223> Ser

<220>
<221> VARIANT
<222> (16)..(16)
<223> Gly

<400> 12

Gly Gln Gly Gly Gln Gly Gln Gly Gly Tyr Gly Gln Gly Ala Gly Ser
1 5 10 15

Ser

<210> 13
<211> 14
<212> PRT
<213> Artificial Sequence

<220>
<223> Consensus sequence derived from internal repeats of Euprosthenops australis MaSp1

<220>
<221> VARIANT
<222> (2)..(2)
<223> Gln

<220>
<221> VARIANT
<222> (6)..(6)
<223> Arg

<220>
<221> VARIANT
<222> (11)..(11)
<223> Ser

<220>
<221> VARIANT
<222> (11)..(11)
<223> Val

<400> 13

Gly Arg Gly Gln Gly Gly Tyr Gly Gln Gly Ala Gly Gly Asn
1 5 10

<210> 14
<211> 888
<212> DNA
<213> Euprosthenops australis

<400> 14
ggttctggga attcacacac tacaccatgg acaaacccag gactcgcaga aaacttcatg 60

aacagtttca tgcaaggcct gagctcgatg ccaggtttca cggcaagcca attggatgat 120

atgtcaacca tcgcacaatc catggtacag tcaatacaat ccttggcggc acaaggcagg 180

acatcaccga ataagctgca ggcccttaac atggcttttg catcttcgat ggcagaaatc 240

gcggcatccg aagaaggagg gggaagcctt tccaccaaaa ctagctctat agccagtgca 300

atgtccaacg cgtttctgca aacaactgga gtggtaaacc aaccgttcat aaatgaaata 360

actcagctcg ttagcatgtt tgctcaagca ggtatgaatg atgtcagtgc ttccgcatca 420
gcaggagcat ccgcagcagc atccgcagga gcggctagcg gtcaaggtgg atatggtgga 480
ctaggtcaag gaggatatgg acaaggtgca ggaagttctg cagccgctgc cgccgccgca 540
gcagccgccg cagcaggtgg acaaggtgga caaggtcaag gaggatatgg acaaggttca 600
ggaggttctg cagccgccgc cgccgccgca gcagcagcag cagctgcagc agctggacga 660
ggtcaaggag gatatggtca aggttctgga ggtaatgctg ctgccgcagc cgctgccgcc 720
gccgccgccg ctgcagcagc cggacaggga ggtcaaggtg gatatggtag acaaagccaa 780
ggtgctggtt ccgctgctgc tgctgctgct gctgctgccg ctgctgctgc tgcaggatct 840
ggacaaggtg gatacggtgg acaaggtcaa ggaggttatg gtcagagt 888

<210> 15
<211> 1272
<212> DNA
<213> Euprosthenops australis

<400> 15
atgaaagcat cacacactac accatggaca aacccaggac tcgcagaaaa cttcatgaac 60
agtttcatgc aaggcctgag ctcgatgcca ggtttcacgg caagccaatt ggatgatatg 120
tcaaccatcg cacaatccat ggtacagtca atacaatcct tggcggcaca aggcaggaca 180
tcaccgaata agctgcaggc ccttaacatg gcttttgcat cttcgatggc agaaatcgcg 240
gcatccgaag aaggaggggg aagcctttcc accaaaacta gctctatagc cagtgcaatg 300
tccaacgcgt ttctgcaaac aactggagtg gtaaaccaac cgttcataaa tgaaataact 360
cagctcgtta gcatgtttgc tcaagcaggt atgaatgatg tcagtgcttc cgcatcagca 420
ggagcatccg cagcagcatc cgcaggagcg gctagcggtc aaggtggata tggtggacta 480
ggtcaaggag gatatggaca aggtgcagga agttctgcag ccgctgccgc cgccgcagca 540
gccgccgcag caggtggaca aggtggacaa ggtcaaggag gatatggaca aggttcagga 600
ggttctgcag ccgccgccgc cgccgcagca gcagcagcag ctgcagcagc tggacgaggt 660
caaggaggat atggtcaagg ttctggaggt aatgctgctg ccgcagccgc tgccgccgcc 720
gccgccgctg cagcagccgg acagggaggt caaggtggat atggtagaca aagccaaggt 780
gctggttccg ctgctgctgc tgctgctgct gctgccgctg ctgctgctgc aggatctgga 840
caaggtggat acggtggaca aggtcaagga ggttatggtc agagtagtgc ttctgcttca 900
gctgctgcgt cagctgctag tactgtagct aattcggtga gtcgcctctc atcgccttcc 960
gcagtatctc gagtttcttc agcagtttct agcttggttt caaatggtca agtgaatatg 1020
gcagcgttac ctaatatcat ttccaacatt tcttcttctg tcagtgcatc tgctcctggt 1080
gcttctggat gtgaggtcat agtgcaagct ctactcgaag tcatcactgc tcttgttcaa 1140
atcgttagtt cttctagtgt tggatatatt aatccatctg ctgtgaacca aattactaat 1200
gttgttgcta atgccatggc tcaagtaatg ggcaagcttg cggccgcact cgagcaccac 1260
caccaccacc ac 1272

```
<210>  16
<211>  1020
<212>  DNA
<213>  Euprosthenops australis

<400>  16
ggttctggga attcacacac tacaccatgg acaaacccag gactcgcaga aaacttcatg      60

aacagtttca tgcaaggcct gagctcgatg ccaggtttca cggcaagcca attggatgat     120

atgtcaacca tcgcacaatc catggtacag tcaatacaat ccttggcggc acaaggcagg     180

acatcaccga ataagctgca ggcccttaac atggcttttg catcttcgat ggcagaaatc     240

gcggcatccg aagaaggagg gggaagcctt tccaccaaaa ctagctctat agccagtgca     300

atgtccaacg cgtttctgca aacaactgga gtggtaaacc aaccgttcat aaatgaaata     360

actcagctcg ttagcatgtt tgctcaagca ggtatgaatg atgtcagtgc ttccgcatca     420

gcaggagcat ccgcagcagc atccgcagga gcgccaggtt acagtcctgc accaagctac     480

agttcgggag gttatgcttc aagtgctgcc tcagcagccg ctgcagcagg acaaggagga     540

cctgggggat acggtccagc acctaaccaa ggagcttcat ctgccgctgc tgcagccgca     600

ggatcaggac aaggaccatc aggaccgtac ggtacatctt accagataag tacacaatat     660

actcaaacaa cgacttcaca gggacaagga tatgggtcaa gtagcgctgg agccgcagct     720

gcaggcgctg caggtgctgg acaagggggc tacggaggtc aaggtcaagg aggatatggt     780

caaggagccg gaggtgctgc cgcagcagcc gccgctgccg cagccgctgc cgccgcagcc     840

ggacaaggtg gacaaggtgg aggaggatat ggacaaggag gacaaggagg acaaggagga     900

caaggtcaag gaggatatgg acaaggtgca ggaagttctg cagccgccgc cgccgcagca     960

gcagcagccg ccgcagcagc aggacgaggt caaggaggat atggtccagg ttctggaggt    1020
```

## Description

### Technical field of the invention

The present invention relates to the field of recombinant production of proteins, and more specifically to recombinant production of spider silk proteins (spidroins). The present invention provides a method of producing polymers of an isolated spider silk protein. There is also provided novel spider silk proteins and methods and polynucleic acid molecules for producing such proteins and polymers thereof.

### Background to the invention

Spider silks are nature's high-performance polymers, obtaining extraordinary toughness due to a combination of strength and elasticity. Spiders have up to seven different glands which produce a variety of silk types with different mechanical properties and functions. Dragline silk, produced by the major ampullate gland, is the toughest fiber, and on a weight basis it outperforms man-made materials, such as tensile steel. The properties of dragline silk are attractive in development of new materials for medical or technical purposes.

Dragline silk consists of two main polypeptides, mostly referred to as major ampullate spidroin (MaSp) 1 and 2, but e.g. as ADF-3 and ADF-4 in *Araneus diadematus.* These proteins have molecular masses in the range of 200-720 kDa. The genes coding for dragline proteins of *Latrodectus hesperus* are the only ones that have been completely characterised, and the MaSp1 and MaSp2 genes encode 3129 and 3779 amino acids, respectively (Ayoub NA et al. PLoS ONE 2(6): e514, 2007). The properties of dragline silk polypeptides are discussed in Huemmerich, D. et al. Curr. Biol. 14, 2070-2074 (2004).

Spider dragline silk proteins, or MaSps, have a tripartite composition; a non-repetitive N-terminal domain, a central repetitive region comprised of many iterated poly-Ala/Gly segments, and a non-repetitive C-terminal domain. It is generally believed that the repetitive region forms intermolecular contacts in the silk fibers, while the precise functions of the terminal domains are less clear. It is also believed that in association with fiber formation, the repetitive region undergoes a structural conversion from random coil and $\alpha$-helical conformation to $\beta$-sheet structure. The C-terminal region of spidroins is generally conserved between spider species and silk types. The N-terminal domain of spider silks is the most conserved region (Rising, A. et al. Biomacromolecules 7, 3120-3124 (2006)), but its function is not understood.

Spider silk proteins and fragments thereof are difficult to produce recombinantly in soluble form. Most previous attempts to produce artificial spider silk fibers have included solubilization steps in non-physiological solvents. Several factors complicate the expression of dragline silk proteins. Due to the highly repetitive nature of the genes, and the concomitant restricted amino acid composition of the proteins, transcription and translation errors occur. Depletion of tRNA pools in microbial expression systems, with subsequent discontinuous translation, leading to premature termination of protein synthesis might be another reason. Other reasons discussed for truncation of protein synthesis are secondary structure formation of the mRNA, and recombination of the genes. Native MaSp genes larger than 2.5 kb have been shown to be instable in bacterial hosts. Additionally, there are difficulties in maintaining the recombinant silk proteins in soluble form, since both natural-derived dragline silk fragments and designed block co-polymers, especially MaSp1/ADF-4-derived proteins, easily self-assemble into amorphous aggregates, causing precipitation and loss of protein. See Huemmerich, D. et al. Biochemistry 43, 13604-13612 (2004) and Lazaris, A. et al. Science 295, 472-476 (2002).

Attempts to produce artificial spider silks have employed natural or synthetic gene fragments encoding dragline silk proteins. Recombinant dragline silk proteins have been expressed in various systems including bacteria, yeast, mammalian cells, plants, insect cells, transgenic silkworms and transgenic goats. See e.g. Lewis, R.V. et al. Protein Expr. Purif. 7, 400-406 (1996); Fahnestock, S. R. & Irwin, S. L. Appl. Microbiol. Biotechnol. 47, 23-32 (1997); Arcidiacono, S. et al. Appl. Microbiol. Biotechnol. 49, 31-38 (1998); Fahnestock, S. R. & Bedzyk, L. A. Appl. Microbiol. Biotechnol. 47, 33-39 (1997); and Lazaris, A. et al. Science 295, 472-476 (2002).

Huemmerich, D. et al. Biochemistry 43, 13604-13612 (2004) discloses a synthetic gene, "$(AQ)_{12}NR_3$", coding for repetitive Ala-rich and Gly/Gln-rich fragments and a non-repetitive fragment, all derived from ADF3 from Araneus. The gene is expressed into a soluble protein which aggregates but does not form polymers or fibers.

WO 03/057727 discloses expression of soluble recombinant silk polypeptides in mammalian cell lines and animals. The obtained silk polypeptides exhibit poor solubility in aqueous media and/or form precipitates. Since the obtained silk polypeptides do not polymerise spontaneously, spinning is required to obtain polymers or fibers.

WO 07/078239 and Stark, M. et al. Biomacromolecules 8, 1695-1701, (2007) disclose a miniature spider silk protein consisting of a repetitive fragment with a high content of Ala and Gly and a C-terminal fragment of a protein, as well as soluble fusion proteins comprising the spider silk protein. Fibers of the spider silk protein are obtained spontaneously upon liberation of the spider silk protein from its fusion partner. The small fusion unit is sufficient and necessary for the fiber formation.

Hedhammar, M. et al. Biochemistry 47, 3407-3417, (2008) studies the thermal, pH and salt effects on the structure and aggregation and/or polymerisation of recombinant N- and C-terminal spidroin domains and a repetitive spidroin domain containing four poly-Ala and Gly rich co-blocks.

Summary of the invention

It is an object of the present invention to provide a method of producing polymers of spider silk proteins, wherein spider silk protein solubility and polymerisation is controlled.

It is also an object of the present invention to provide a method of producing fibers of spider silk proteins, wherein spider silk protein solubility and fiber formation is controlled.

It is another object of the present invention to provide a novel spider silk protein, which can provide spider silk fibers, films, foams, nets and meshes.

It is one object of the present invention to provide a water-soluble spider silk protein, which can readily be manipulated to polymerise into fibers at wish. This property allows for all the following steps to be undertaken under physiological conditions, which decreases the risk for toxicity and protein denaturation.

It is yet another object of the present invention to provide fibers of a novel spider silk protein.

It is one object of the present invention to provide spider silk proteins in large scale, which can readily be manipulated to polymerise into fibers at wish.

It is also an object of the invention to provide methods of producing spider silk proteins and fibers of spider silk proteins.

For these and other objects that will be evident from the following disclosure, the present invention provides according to a first aspect a method of producing polymers of an isolated spider silk protein, comprising the steps of:

(i) providing a spider silk protein consisting of from 170 to 600 amino acid residues and comprising:

an N-terminal fragment of from 100 to 160 amino acid residues derived from the N-terminal fragment of a spider silk protein; and

a repetitive fragment of from 70 to 300 amino acid residues derived from the repetitive fragment of apidroin protein; and optionally

a C-terminal fragment of from 70 to 120 amino acid residues, which fragment is derived from the C-terminal fragment of a spider silk protein;

(ii) providing a solution of said spider silk protein in a liquid medium at pH 6.4 or higher and/or an ion composition that prevents polymerisation of said spider silk protein, optionally involving removal of lipopolysaccharides and other pyrogens;

(iii) adjusting the properties of said liquid medium to a pH of 6.3 or lower and an ion composition that allows polymerisation of said spider silk protein;

(iv) allowing the spider silk protein to form solid polymers in the liquid medium, said liquid medium having a pH of 6.3 or lower and an ion composition that allows polymerisation of said spider silk protein; and

(v) isolating the solid spider silk protein polymers from said liquid medium.

In one embodiment, the pH of the liquid medium of steps (iii) and (iv) is 6.2 or lower, such as 6.0 or lower. In certain embodiments, the ionic strength of the liquid medium of steps (iii) and (iv) is in the range of 1-250 mM.

In an embodiment, the pH of the liquid medium of step (ii) is 6.7 or higher, such as 7.0 or higher.

According to another aspect, the present invention provides a polymer of a spider silk protein, said protein consisting of from 170 to 600 amino acid residues and comprising:

an N-terminal fragment of from 100 to 160 amino acid residues derived from the N-terminal fragment of a spider silk protein; and

a repetitive fragment of from 70 to 300 amino acid residues derived from the repetitive fragment of a spider silk protein; and optionally

a C-terminal fragment of from 70 to 120 amino acid residues, which fragment is derived from the C-terminal fragment of a spider silk protein.

In preferred embodiments of these two aspects, the protein is selected from the group of proteins defined by the formulas **NT-REP-CT** and **NT-REP,** wherein

**NT** is a protein fragment having from 100 to 160 amino acid residues, which fragment is a N-terminal fragment derived from a spider silk protein.

**REP** is a protein fragment having from 70 to 300 amino acid residues, wherein said fragment is selected from the group of **L**(**AG**)$_n$**L**, **L**(**AG**)$_n$**AL**, **L**(**GA**)$_n$**L**, **L**(**GA**)$_n$**GL**, wherein
n is an integer from 2 to 8;
each individual **A** segment is an amino acid sequence of from 8 to 18 amino acid residues, wherein from 0 to 3 of the amino acid residues are not Ala, and the remaining amino acid residues are Ala;
each individual **G** segment is an amino acid sequence of from 12 to 30 amino acid residues, wherein at least 40% of the amino acid residues are Gly; and each individual **L** segment is a linker amino acid sequence of from 0 to 20 amino acid residues; and
**CT** is a protein fragment having from 70 to 120 amino acid residues, which fragment is a C-terminal fragment derived from a spider silk protein.

In one embodiment, the polymer is a fiber, film, foam, net or mesh. In a preferred embodiment, the polymer is a fiber having a diameter of more than 0.1 μm and a length of more than 5 mm.

According to one aspect, the present invention provides a method of producing dimers of an isolated spider silk protein, comprising the steps of:

(i) providing a spider silk protein of from 170 to 600 amino acid residues, said protein comprising:

an N-terminal fragment of from 100 to 160 amino acid residues derived from the N-terminal fragment of a spider silk protein; and
a repetitive fragment of from 70 to 300 amino acid residues derived from the repetitive fragment of a spider silk protein; and optionally a C-terminal fragment of from 70 to 120 amino acid residues, which fragment is derived from the C-terminal fragment of a spider silk protein;

(ii) providing a solution of dimers of the spider silk protein in a liquid medium at a pH of 6.4 or higher and/or an ion composition that prevents polymerisation of said spider silk protein; and
(iii) isolating the dimers obtained in step (ii), optionally involving removal of lipopolysaccharides and other pyrogens.

According to an aspect, the present invention provides a dimer of a spider silk protein, said protein consisting of from 170 to 600 amino acid residues and comprising:

an N-terminal fragment of from 100 to 160 amino acid residues derived from the N-terminal fragment of a spider silk protein; and
a repetitive fragment of from 70 to 300 amino acid residues derived from the repetitive fragment of a spider silk protein; and optionally
a C-terminal fragment of from 70 to 120 amino acid residues, which fragment is derived from the C-terminal fragment of a spider silk protein.

According to another aspect, the present invention provides an isolated spider silk protein, which consists of from 170 to 600 amino acid residues and is selected from the group of proteins defined by the formulas **NT-REP-CT** and **NT-REP,** wherein
**NT** is a protein fragment having from 100 to 160 amino acid residues, which fragment is a N-terminal fragment derived from a spider silk protein.

**REP** is a protein fragment having from 70 to 300 amino acid residues, wherein said fragment is selected from the group of **L**(**AG**)$_n$**L**, **L**(**AG**)$_n$**AL**, **L**(**GA**)$_n$**L**, **L**(**GA**)$_n$**GL**, wherein
n is an integer from 2 to 8;
each individual **A** segment is an amino acid sequence of from 8 to 18 amino acid residues, wherein from 0 to 3 of the amino acid residues are not Ala, and the remaining amino acid residues are Ala;
each individual **G** segment is an amino acid sequence of from 12 to 30 amino acid residues, wherein at least 40% of the amino acid residues are Gly; and each individual L segment is a linker amino acid sequence of from 0 to 20 amino acid residues; and
**CT** is a protein fragment having from 70 to 120 amino acid residues, which fragment is a C-terminal fragment derived from a spider silk protein.

According to one aspect, the present invention provides use of the spider silk proteins of the inventions for producing polymers of the spider silk protein.

According to one aspect, the present invention provides a composition comprising an isolated spider silk protein according to the invention dissolved in a liquid medium having a pH of 6.4 or higher and/or an ion composition that prevents polymerisation of said spider silk protein.

According to another aspect, the present invention provides an isolated polynucleic acid molecule comprising a nucleic acid sequence selected from the group consisting of SEQ ID NO: 14-16; nucleic acid sequences encoding SEQ ID NO: 3-5; nucleic acid sequences which encodes a spider silk protein according to the invention; and their complementary nucleic acid sequences.

According to yet another aspect, the present invention provides a method of producing a spider silk protein according to the invention, comprising the steps of:

(i) expressing a polynucleic acid molecule which encodes said spider silk protein in a suitable host; and
(ii) isolating the protein obtained in step (i), optionally involving removal of lipopolysaccharides and other pyrogens.

Furthermore, there is provided a method of reversibly assembling a polymer or oligomer of one type of molecule or several different types of molecules, comprising the steps of:

(i) providing said molecules, each molecule comprising

(a) a first binding moiety of from 100 to 160 amino acid residues which is derived from the N-terminal fragment of a spider silk protein, and
(b) a second moiety which is individually selected from proteins, nucleic acids, carbohydrates and lipids;

(ii) providing a solution of said molecules in a liquid medium at pH 6.4 or higher and/or an ion composition that prevents polymerisation or oligomerisation of said molecule(s) via said binding moieties;
(iii) adjusting the properties of said liquid medium to a pH of 6.3 or lower and an ion composition that allows polymerisation or oligomerisation of said molecules via said binding moieties;
(iv) allowing said molecules to assemble into a polymer or oligomer via said binding moieties in the liquid medium, said liquid medium having a pH of 6.3 or lower and an ion composition that allows polymerisation or oligomerisation of said molecules via said binding moieties.

There is also provided a method of detecting binding interactions between a subset of molecules comprised in a set of molecules, comprising the steps of:

(i) providing said set of molecules, each molecule comprising

(a) a first binding moiety of from 100 to 160 amino acid residues which is derived from the N-terminal fragment of a spider silk protein, and
(b) a second moiety which is individually selected from proteins, nucleic acids, carbohydrates and lipids;

(ii) providing a solution of said set of molecules in a liquid medium at pH 6.4 or higher and/or an ion composition that prevents polymerisation or oligomerisation of said molecules;
(iii) adjusting the properties of said liquid medium to a pH of 6.3 or lower and an ion composition that allows polymerisation or oligomerisation of said molecules;
(iv) allowing said molecules to assemble into a polymer or oligomer via said binding moieties in the liquid medium, said liquid medium having a pH of 6.3 or lower and an ion composition that allows polymerisation or oligomerisation of said molecules;
(v) adjusting the properties of said liquid medium to a pH of 6.4 or higher and/or an ion composition that prevents polymerisation or oligomerisation of said molecules to disassemble said polymer or oligomer; and
(vi) determining the presence of binding interactions which are not mediated *via* said binding moieties between two or more different molecules, which form said subset of molecules.

There is also provided a novel use of one or more molecules, each comprising

(a) a first binding moiety of from 100 to 160 amino acid residues which is derived from the N-terminal fragment of a spider silk protein, and
(b) a second moiety which is individually selected from proteins, nucleic acids, carbohydrates and lipids;

for reversibly assembling a polymer or oligomer of said molecules via said binding moieties in a solution at a pH of 6.3 or lower and an ion composition that allows polymerisation or oligomerisation of said spider silk protein.

Other aspects and embodiments of the invention will be evident from the description, including the itemised listing of embodiments.

List of appended sequences

| SEQ ID NO | |
|---|---|
| 1 | 4Rep |
| 2 | 4RepCT |
| 3 | NT4Rep |
| 4 | NT5Rep |
| 5 | NT4RepCTHis |
| 6 | NT |
| 7 | CT |
| 8 | consensus NT sequence |
| 9 | consensus CT sequence |
| 10 | repetitive sequence from *Euprosthenops australis* MaSp1 |
| 11 | consensus G segment sequence 1 |
| 12 | consensus G segment sequence 2 |
| 13 | consensus G segment sequence 3 |
| 14 | NT4Rep (DNA) |
| 15 | NT4RepCT (DNA) |
| 16 | NT5Rep (DNA) |

Brief description of the drawings

Fig 1 shows a sequence alignment of spidroin N-terminal domains.
Fig 2 shows a sequence alignment of spidroin C-terminal domains.
Fig 3 illustrates the pH-induced and salt-dependent polymerisation of NT4Rep, NT4RepCT, 4Rep, and 4RepCT.
Fig 4 shows turbidimetry of NT and NT4Rep at different pHs.
Fig 5 shows dynamic light scattering of NT at pH 6 and 7.
Fig 6 shows dynamic light scattering of NT at pH 6.1-7.2 in various ion compositions.
Fig 7 schematically shows pH dependent assembly of NT-fusion proteins.

Itemized listing of embodiments

1. A method of producing polymers of an isolated spider silk protein, comprising the steps of:

(i) providing a spider silk protein consisting of from 170 to 600 amino acid residues and comprising:

an N-terminal fragment of from 100 to 160 amino acid residues derived from the N-terminal fragment of a spider silk protein; and
a repetitive fragment of from 70 to 300 amino acid residues derived from the repetitive fragment of a spider silk protein; and optionally
a C-terminal fragment of from 70 to 120 amino acid residues, which fragment is derived from the C-terminal fragment of a spider silk protein;

(ii) providing a solution of said spider silk protein in a liquid medium at pH 6.4 or higher and/or an ion composition that prevents polymerisation of said spider silk protein, optionally involving removal of lipopolysaccharides and other pyrogens;
(iii) adjusting the properties of said liquid medium to a pH of 6.3 or lower and an ion composition that allows polymerisation of said spider silk protein;
(iv) allowing the spider silk protein to form solid polymers in the liquid medium, said liquid medium having a pH of 6.3 or lower and an ion composition that allows polymerisation of said spider silk protein; and
(v) isolating the solid spider silk protein polymers from said liquid medium.

2. A method according to item 1, wherein the pH of the liquid medium of steps (iii) and (iv) is 6.2 or lower, such as 6.0 or lower.
3. A method according to any one of items 1-2, wherein the ionic strength of the liquid medium of steps (iii) and (iv) is in the range of 1-250 mM.
4. A method according to any one of items 1-3, wherein the pH of the liquid medium of step (ii) is 6.7 or higher, such as 7.0 or higher.
5. A method according to any one of items 1-3, wherein the pH of the liquid medium of step (ii) is in the range of 6.4-6.8.
6. A method according to any one of items 1-5, wherein said polymer is a fiber, film, foam, net or mesh.
7. A method according to item 5, wherein said polymer is a fiber having a diameter of more than 0.1 μm and a length of more than 5 mm.
8. A method of producing dimers of an isolated spider silk protein, comprising the steps of:

(i) providing a spider silk protein of from 170 to 600 amino acid residues, said protein comprising:

an N-terminal fragment of from 100 to 160 amino acid residues derived from the N-terminal fragment of a spider silk protein; and
a repetitive fragment of from 70 to 300 amino acid residues derived from the repetitive fragment of a spider silk protein; and optionally
a C-terminal fragment of from 70 to 120 amino acid residues, which fragment is derived from the C-terminal fragment of a spider silk protein;

(ii) providing a solution of dimers of the spider silk protein in a liquid medium at a pH of 6.4 or higher and/or an ion composition that prevents polymerisation of said spider silk protein; and
(iii) isolating the dimers obtained in step (ii), optionally involving removal of lipopolysaccharides and other pyrogens.

9. A method according to item 8, wherein the pH of the liquid medium of step (ii) is 6.7 or higher, such as 7.0 or higher.
10. A method according to any one of items 1-9, wherein step (i) of providing said spider silk protein is comprising the sub-steps of:

(a) expressing a polynucleic acid molecule which encodes said spider silk protein in a suitable host; and
(b) isolating the protein obtained in sub-step (a), optionally involving removal of lipopolysaccharides and other pyrogens.

11. A method according to any one of items 1-10, wherein said protein is selected from the group of proteins defined by the formulas NT-REP-CT and NT-REP, wherein
NT is a protein fragment having from 100 to 160 amino acid residues, which fragment is a N-terminal fragment derived from a spider silk protein.
REP is a protein fragment having from 70 to 300 amino acid residues, wherein said fragment is selected from the group of $L(AG)_nL$, $L(AG)_nAL$, $L(GA)_nL$, $L(GA)_nGL$, wherein
n is an integer from 2 to 8;
each individual A segment is an amino acid sequence of from 8 to 18 amino acid residues, wherein from 0 to 3 of the amino acid residues are not Ala, and the remaining amino acid residues are Ala;
each individual G segment is an amino acid sequence of from 12 to 30 amino acid residues, wherein at least 40% of the amino acid residues are Gly; and each individual L segment is a linker amino acid sequence of from 0 to 20 amino acid residues; and
CT is a protein fragment having from 70 to 120 amino acid residues, which fragment is a C-terminal fragment derived from a spider silk protein.
12. A polymer of a spider silk protein, said protein consisting of from 170 to 600 amino acid residues and comprising:

an N-terminal fragment of from 100 to 160 amino acid residues derived from the N-terminal fragment of a spider silk protein; and
a repetitive fragment of from 70 to 300 amino acid residues derived from the repetitive fragment of a spider silk protein; and optionally
a C-terminal fragment of from 70 to 120 amino acid residues, which fragment is derived from the C-terminal fragment of a spider silk protein.

13. A polymer of a spider silk protein according to item 12, wherein said protein is selected from the group of proteins defined by the formulas NT-REP-CT and NT-REP, wherein
NT is a protein fragment having from 100 to 160 amino acid residues, which fragment is a N-terminal fragment derived from a spider silk protein.
REP is a protein fragment having from 70 to 300 amino acid residues, wherein said fragment is selected from the group of $L(AG)_nL$, $L(AG)_nAL$, $L(GA)_nL$, $L(GA)_nGL$, wherein
n is an integer from 2 to 8;
each individual A segment is an amino acid sequence of from 8 to 18 amino acid residues, wherein from 0 to 3 of the amino acid residues are not Ala, and the remaining amino acid residues are Ala;
each individual G segment is an amino acid sequence of from 12 to 30 amino acid residues, wherein at least 40% of the amino acid residues are Gly; and each individual L segment is a linker amino acid sequence of from 0 to 20 amino acid residues; and
CT is a protein fragment having from 70 to 120 amino acid residues, which fragment is a C-terminal fragment derived from a spider silk protein.
14. A polymer of a spider silk protein according to any one of items 12-13, wherein said polymer consists of polymerised dimers of said protein.
15. A polymer of a spider silk protein according to any one of items 12-14, wherein the content of lipopolysaccharides and other pyrogens is 1 EU/mg of isolated protein or lower.
16. A polymer of a spider silk protein according to any one of items 12-15, wherein said polymer is a fiber, film, foam, net or mesh.
17. A polymer of a spider silk protein according to item 16, wherein said polymer is a fiber having a diameter of more than 0.1 μm and a length of more than 5 mm.
18. A dimer of a spider silk protein, said protein consisting of from 170 to 600 amino acid residues and comprising:

an N-terminal fragment of from 100 to 160 amino acid residues derived from the N-terminal fragment of a spider silk protein; and
a repetitive fragment of from 70 to 300 amino acid residues derived from the repetitive fragment of a spider silk protein; and optionally
a C-terminal fragment of from 70 to 120 amino acid residues, which fragment is derived from the C-terminal fragment of a spider silk protein.

19. A dimer of a spider silk protein according to item 18, wherein said protein is selected from the group of proteins defined by the formulas NT-REP-CT and NT-REP, wherein
NT is a protein fragment having from 100 to 160 amino acid residues, which fragment is a N-terminal fragment derived from a spider silk protein.
REP is a protein fragment having from 70 to 300 amino acid residues, wherein said fragment is selected from the group of $L(AG)_nL$, $L(AG)_nAL$, $L(GA)_nL$, $L(GA)_nGL$, wherein
n is an integer from 2 to 8;
each individual A segment is an amino acid sequence of from 8 to 18 amino acid residues, wherein from 0 to 3 of the amino acid residues are not Ala, and the remaining amino acid residues are Ala;
each individual G segment is an amino acid sequence of from 12 to 30 amino acid residues, wherein at least 40% of the amino acid residues are Gly; and each individual L segment is a linker amino acid sequence of from 0 to 20 amino acid residues; and
CT is a protein fragment having from 70 to 120 amino acid residues, which fragment is a C-terminal fragment derived from a spider silk protein.
20. An isolated spider silk protein, which consists of from 170 to 600 amino acid residues and is selected from the group of proteins defined by the formulas NT-REP-CT and NT-REP, wherein
NT is a protein fragment having from 100 to 160 amino acid residues, which fragment is a N-terminal fragment derived from a spider silk protein.
REP is a protein fragment having from 70 to 300 amino acid residues, wherein said fragment is selected from the group of $L(AG)_nL$, $L(AG)_nAL$, $L(GA)_nL$, $L(GA)_nGL$, wherein
n is an integer from 2 to 8;
each individual A segment is an amino acid sequence of from 8 to 18 amino acid residues, wherein from 0 to 3 of the amino acid residues are not Ala, and the remaining amino acid residues are Ala;
each individual G segment is an amino acid sequence of from 12 to 30 amino acid residues, wherein at least 40% of the amino acid residues are Gly; and each individual L segment is a linker amino acid sequence of from 0 to 20 amino acid residues; and
CT is a protein fragment having from 70 to 120 amino acid residues, which fragment is a C-terminal fragment derived from a spider silk protein.

21. A composition comprising an isolated spider silk protein according to item 20 dissolved in a liquid medium having a pH of 6.4 or higher and/or an ion composition that prevents polymerisation of said spider silk protein.
22. A composition according to item 21, said liquid medium having a pH of 7.0 or higher.
23. A composition according to any one of items 21-22, said liquid medium having a pH of 7.0 or higher, wherein the content of lipopolysaccharides and other pyrogens is 1 EU/mg of isolated protein or lower.
24. Use of a spider silk protein according to item 20 for producing dimers of the spider silk protein.
25. Use of a spider silk protein according to item 20 for producing polymers of the spider silk protein.
26. Use of a dimer of a spider silk protein according to any one of items 18-19 for producing polymers of the isolated spider silk protein.
27. Use according to any one of items 25-26, wherein said polymers are produced in a liquid medium having a pH of 6.3 or lower and an ion composition that allows polymerisation of said spider silk protein.
28. An isolated polynucleic acid molecule comprising a nucleic acid sequence selected from the group consisting of SEQ ID NO: 14-16; nucleic acid sequences encoding SEQ ID NO: 3-5; nucleic acid sequences which encodes a spider silk protein according to item 20; and their complementary nucleic acid sequences.
29. A method of producing a spider silk protein according to item 20, comprising the steps of:

(i) expressing a polynucleic acid molecule which encodes said spider silk protein in a suitable host; and
(ii) isolating the protein obtained in step (i), optionally involving removal of lipopolysaccharides and other pyrogens.

30. A method of reversibly assembling a polymer or oligomer of one type of molecule or several different types of molecules, comprising the steps of:

(i) providing said molecules, each molecule comprising

(a) a first binding moiety of from 100 to 160 amino acid residues which is derived from the N-terminal fragment of a spider silk protein, and
(b) a second moiety which is individually selected from proteins, nucleic acids, carbohydrates and lipids;

(ii) providing a solution of said molecules in a liquid medium at pH 6.4 or higher and/or an ion composition that prevents polymerisation or oligomerisation of said molecule(s) via said binding moieties;
(iii) adjusting the properties of said liquid medium to a pH of 6.3 or lower and an ion composition that allows polymerisation or oligomerisation of said molecules via said binding moieties;
(iv) allowing said molecules to assemble into a polymer or oligomer via said binding moieties in the liquid medium, said liquid medium having a pH of 6.3 or lower and an ion composition that allows polymerisation or oligomerisation of said molecules via said binding moieties.

31. A method according to item 30, wherein said molecules of step (i) are identical, and wherein said polymer or oligomer of step (iv) is a homopolymer or a homooligomer.
32. A method according to item 30, wherein said molecules of step (i) are not identical, and wherein said polymer or oligomer of step (iv) is a heteropolymer or heterooligomer.
33. A method according to any one of items 30-32, wherein said polymer or oligomer of step (iv) is dissolved in said liquid medium having a pH of 6.3 or lower and an ion composition that allows polymerisation or oligomerisation of said molecules.
34. A method according to any one of items 30-33, wherein the pH of the liquid medium of steps (iii) and (iv) is 6.2 or lower, such as 6.0 or lower.
35. A method according to any one of items 30-34, wherein the ionic strength of the liquid medium of step (iv) is in the range of 1-250 mM.
36. A method according to any one of items 30-35, wherein the pH of the liquid medium of step (ii) is 6.7 or higher, such as 7.0 or higher.
37. A method according to any one of items 30-35, wherein the pH of the liquid medium of step (ii) is in the range of 6.4-6.8.
38. A method according to any one of items 30-37, wherein said second moiety is a protein.
39. A method according to any one of items 30-38, further comprising the step of:

(v) adjusting the properties of said liquid medium to a pH of 6.4 or higher and/or an ion composition that prevents polymerisation or oligomerisation of said molecules to disassemble said polymer or oligomer.

40. A method according to item 39, wherein the pH of the liquid medium of step (v) is 6.7 or higher, such as 7.0 or higher.
41. A method according to item 39, wherein the pH of the liquid medium of step (v) is in the range of 6.4-6.8.
42. A method according to any one of items 39-41, wherein the polymer or oligomer of step (iv) is used in interaction studies, separation, inducing activity of co-enzyme complexes or FRET analysis.
43. A method according to any one of items 30-42, wherein at least one molecule type of step (i) is immobilised to a solid support.
44. A method of detecting binding interactions between a subset of molecules comprised in a set of molecules, comprising the steps of:

(i) providing said set of molecules, each molecule comprising

(a) a first binding moiety of from 100 to 160 amino acid residues which is derived from the N-terminal fragment of a spider silk protein, and
(b) a second moiety which is individually selected from proteins, nucleic acids, carbohydrates and lipids;

(ii) providing a solution of said set of molecules in a liquid medium at pH 6.4 or higher and/or an ion composition that prevents polymerisation or oligomerisation of said molecules;
(iii) adjusting the properties of said liquid medium to a pH of 6.3 or lower and an ion composition that allows polymerisation or oligomerisation of said molecules;
(iv) allowing said molecules to assemble into a polymer or oligomer via said binding moieties in the liquid medium, said liquid medium having a pH of 6.3 or lower and an ion composition that allows polymerisation or oligomerisation of said molecules;
(v) adjusting the properties of said liquid medium to a pH of 6.4 or higher and/or an ion composition that prevents polymerisation or oligomerisation of said molecules to disassemble said polymer or oligomer; and
(vi) determining the presence of binding interactions which are not mediated *via* said binding moieties between two or more different molecules, which form said subset of molecules.

45. Use of one or more molecules, each comprising

(a) a first binding moiety of from 100 to 160 amino acid residues which is derived from the N-terminal fragment of a spider silk protein, and
(b) a second moiety which is individually selected from proteins, nucleic acids, carbohydrates and lipids;

for reversibly assembling a polymer or oligomer of said molecules via said binding moieties in a solution at a pH of 6.3 or lower and an ion composition that allows polymerisation or oligomerisation of said molecules.
46. Use according to item 45, wherein the polymer or oligomer is used in interaction studies, separation, inducing activity of co-enzyme complexes or FRET analysis.

Detailed description of the invention

The present invention is generally based on the inventive insight that the previously poorly understood N-terminal non-repetitive fragment of spider silk proteins is involved in polymerisation of these proteins, and in particular that the formation of polymers involving this fragment can be tightly controlled by varying certain parameters. This insight has been developed into a novel method of producing polymers of an isolated spider silk protein. Although the examples by necessity relate to specific proteins, in this case proteins derived from major spidroin 1 (MaSp1) from *Euprosthenops australis,* it is considered that the method disclosed herein is applicable to any similar protein for the purpose of producing polymers.
This insight has also led to the identification of a novel spider silk protein motif, which is sufficient for recombinant production of spider silk fibers. It follows that the new spider silk protein motif is useful as a starting point for construction of novel spider silk proteins and genes, such as those reported herein. The polymers which are formed from the proteins resulting from the novel spidroins are useful for their physical properties, especially the useful combination of high strength, elasticity and light weight. They are also useful for their ability to support cell adherence and growth. The properties of dragline silk are attractive in development of new materials for medical or technical purposes. In particular, spider silks according to the invention are useful in medical devices, such as implants and medical products, such as wound closure systems, band-aids, sutures, wound dressings, and scaffolds for tissue engineering and guided cell regeneration. Spider silks according to the invention are also particularly useful for use as textile or fabric, such as in parachutes, bulletproof clothing, seat belts, etc. Using this method, it is no longer required to introduce a cleavable fusion partner that is cleaved off using cleavage agents during the process when polymerisation is desired, This facilitates the production and yield of spider silk proteins and polymers thereof, and provides an advantage in an industrial production process.

The term "fiber" as used herein relates to polymers having a thickness of at least 0.1 μm, preferably macroscopic polymers that are visible to the human eye, i.e. having a thickness of at least 1 μm, and have a considerable extension in length compared to its thickness, preferably above 5 mm. The term "fiber" does not encompass unstructured aggregates or precipitates.

The terms "spidroins" and "spider silk proteins" are used interchangeably throughout the description and encompass all known spider silk proteins, including major ampullate spider silk proteins which typically are abbreviated "MaSp", or "ADF" in the case of *Araneus diadematus.* These major ampullate spider silk proteins are generally of two types, 1 and 2. These terms furthermore include the new proteins according to the invention, as defined in the appended claims and itemized embodiments, and other non-natural proteins with a high degree of identity and/or similarity to the known spider silk proteins.

The present invention thus provides a method of producing polymers of an isolated spider silk protein. In the first step, a spider silk protein is provided. The spider silk protein consists of from 170 to 600 amino acid residues, preferably from 280 to 600 amino acid residues, such as from 300 to 400 amino acid residues, more preferably from 340 to 380 amino acid residues. The small size is advantageous because longer spider silk proteins tend to form amorphous aggregates, which require use of harsh solvents for solubilisation and polymerisation. The spider silk protein comprises an N-terminal fragment (NT) derived from the corresponding part of a spider silk protein, and a repetitive fragment (REP) derived from the corresponding internal fragment of a spider silk protein. Optionally, the spider silk protein comprises a C-terminal fragment (CT) derived from the corresponding fragment of a spider silk protein. Thus, the spidroin can schematically be represented by the formula **NT-REP,** and alternatively **NT-REP-CT.** The protein fragments are covalently coupled, typically *via* a peptide bond. In one embodiment, the spider silk protein consists of the **NT** fragment coupled to the **REP** fragment, which **REP** fragment is optionally coupled to the **CT** fragment.

The **NT** fragment has a high degree of similarity to the N-terminal amino acid sequence of spider silk proteins. As shown in Fig 1, this amino acid sequence is well conserved among various species and spider silk proteins, including MaSp1 and MaSp2. In Fig 1, the following spidroin **NT** fragments are aligned, denoted with GenBank accession entries where applicable:

TABLE 1 - Spidroin **NT** fragments

| Code | Species and spidroin protein | GenBank acc. no. |
|---|---|---|
| Ea MaSp1 | *Euprosthenops australis* MaSp 1 | AM259067 |
| Lg MaSp1 | *Latrodectus geometricus* MaSp 1 | ABY67420 |
| Lh MaSp1 | *Latrodectus hesperus* MaSp 1 | ABY67414 |
| Nc MaSp1 | *Nephila clavipes* MaSp 1 | ACF19411 |
| At MaSp2 | *Argiope trifasciata* MaSp 2 | AAZ15371 |
| Lg MaSp2 | *Latrodectus geometricus* MaSp 2 | ABY67417 |
| Lh MaSp2 | *Latrodectus hesperus* MaSp 2 | ABR68855 |
| Nim MaSp2 | *Nephila inaurata madagascariensis* MaSp 2 | AAZ15322 |
| Nc MaSp2 | *Nephila clavipes* MaSp 2 | ACF19413 |
| Ab CySp1 | *Argiope bruennichi* cylindriform spidroin 1 | BAE86855 |
| Ncl CySp1 | *Nephila clavata* cylindriform spidroin 1 | BAE54451 |
| Lh TuSp1 | *Latrodectus hesperus* tubuliform spidroin | ABD24296 |
| Nc Flag | *Nephila clavipes* flagelliform silk protein | AF027972 |
| Nim Flag | *Nephila inaurata* madagascariensis flagelliform silk protein | AF218623 (translated) |

Only the part corresponding to the N-terminal domain is shown for each sequence, omitting the signal peptide. Nc flag and Nlm flag are translated and edited according to Rising A. et al. Biomacromolecules 7, 3120-3124 (2006)).

It is observed that **NT** has a clear dipole moment as acidic and basic residues are localized in clusters at opposite poles. Without desiring to be limited thereto, it is contemplated that the observed polymerisation of **NT** may involve the formation of linear arrays of **NT** dimers, stacked pole-to-pole with the negative surface of one subunit facing the positive surface of the neighbouring subunit in the next dimer in the array.

It is not critical which specific **NT** fragment is present in spider silk proteins according to the invention, as long as the **NT** fragment is not entirely missing. Thus, the **NT** fragment according to the invention can be selected from any of the amino acid sequences shown in Fig 1 or sequences with a high degree of similarity. A wide variety of N-terminal sequences can be used in the spider silk protein according to the invention. Based on the homologous sequences of Fig 1, the following sequence constitutes a consensus **NT** amino acid sequence:

QANTPWSSPNLADAFINSF(M/L)SA(A/I)SSSGAFSADQLDDMSTIG(D/N/Q)T LMSAMD(N/S/K)MGRSG(K/R) STKSKLQALNMAFASSMAEIAAAESGG(G/Q) SVGVKTNAISDALSSAFYQTTGSVNPQFV(N/S)EIRSLI(G/N)M (F/L)(A/S)QAS ANEV (SEQ ID NO: 8).

The sequence of the **NT** fragment according to the invention has at least 50% identity, preferably at least 60% identity, to the consensus amino acid sequence SEQ ID NO: 8, which is based on the amino acid sequences of Fig 1. In a preferred embodiment, the sequence of the **NT** fragment according to the invention has at least 65% identity, preferably at least 70% identity, to the consensus amino acid sequence SEQ ID NO: 8. In preferred embodiments, the **NT** fragment according to the invention has furthermore 70%, preferably 80%, similarity to the consensus amino acid sequence SEQ ID NO: 8.

A representative **NT** fragment according to the invention is the *Euprosthenops australis* sequence SEQ ID NO: 6. According to a preferred embodiment of the invention, the **NT** fragment has at least 80% identity to SEQ ID NO: 6 or any individual amino acid sequence in Fig 1. In preferred embodiments of the invention, the **NT** fragment has at least 90%, such as at least 95% identity, to SEQ ID NO: 6 or any individual amino acid sequence in Fig 1. In preferred embodiments of the invention, the **NT** fragment is identical to SEQ ID NO: 6 or any individual amino acid sequence in Fig 1, in particular to Ea MaSp1.

The **NT** fragment contains from 100 to 160 amino acid residues. It is preferred that the **NT** fragment contains at least 100, or more than 110, preferably more than 120, amino acid residues. It is also preferred that the **NT** fragment contains at most 160, or less than 140 amino acid residues. A typical **NT** fragment contains approximately 130-140 amino acid residues.

The protein fragment **REP** has a repetitive character, alternating between alanine-rich stretches and glycine-rich stretches. The **REP** fragment generally contains more than 70, such as more than 140, and less than 300, preferably less than 240, such as less than 200, amino acid residues, and can itself be divided into several **L** (linker) segments, **A** (alanine-rich) segments and **G** (glycine-rich) segments, as will be explained in more detail below. Typically, said linker segments, which are optional, are located at the **REP** fragment terminals, while the remaining segments are in turn alanine-rich and glycine-rich. Thus, the **REP** fragment can generally have either of the following structures, wherein n is an integer:

$\mathbf{L}(\mathbf{AG})_n\mathbf{L}$, such as $\mathbf{LA_1G_1A_2G_2A_3G_3A_4G_4A_5G_5L}$;
$\mathbf{L}(\mathbf{AG})_n\mathbf{AL}$, such as $\mathbf{LA_1G_1A_2G_2A_3G_3A_4G_4A_5G_5A_6L}$;
$\mathbf{L}(\mathbf{GA})_n\mathbf{L}$, such as $\mathbf{LG_1A_1G_2A_2G_3A_3G_4A_4G_5A_5L}$; or
$\mathbf{L}(\mathbf{GA})_n\mathbf{GL}$, such as $\mathbf{LG_1A_1G_2A_2G_3A_3G_4A_4G_5A_5G_6L}$.

It follows that it is not critical whether an alanine-rich or a glycine-rich segment is adjacent to the N-terminal or C-terminal linker segments. It is preferred that n is an integer from 2 to 8, preferably from 4 to 8, more preferred from 4 to 6, i.e. n=4, n=5 or n=6.

In preferred embodiments, the alanine content of the **REP** fragment according to the invention is above 20%, preferably above 25%, more preferably above 30%, and below 50%, preferably below 40%, more preferably below 35%. This is advantageous, since it is contemplated that a higher alanine content provides a stiffer and/or stronger and/or less extendible fiber.

In certain embodiments, the **REP** fragment is void of proline residues, i.e. there are no Pro residues in the **REP** fragment.

Now turning to the segments that constitute the **REP** fragment according to the invention, it shall be emphasized that each segment is individual, i.e. any two **A** segments, any two **G** segments or any two **L** segments of a specific **REP** fragment may be identical or may not be identical. Thus, it is not a general feature of the invention that each type of segment is identical within a specific **REP** fragment. Rather, the following disclosure provides the skilled person with guidelines how to design individual segments and gather them into a **REP** fragment, which is a part of a functional spider silk protein according to the invention.

Each individual **A** segment is an amino acid sequence having from 8 to 18 amino acid residues. It is preferred that each individual **A** segment contains from 13 to 15 amino acid residues. It is also possible that a majority, or more than two, of the **A** segments contain from 13 to 15 amino acid residues, and that a minority, such as one or two, of the **A** segments contain from 8 to 18 amino acid residues, such as 8-12 or 16-18 amino acid residues. A vast majority of these amino acid residues are alanine residues. More specifically, from 0 to 3 of the amino acid residues are not alanine residues, and the remaining amino acid residues are alanine residues. Thus, all amino acid residues in each individual **A** segment are alanine residues, with no exception or the exception of one, two or three amino acid residues, which can be any amino acid. It is preferred that the alanine-replacing amino acid(s) is (are) natural amino acids, preferably individually selected from the group of serine, glutamic acid, cysteine and glycine, more preferably serine. Of course, it is possible that one or more of the **A** segments are all-alanine segments, while the remaining **A** segments contain 1-3 non-alanine residues, such as serine, glutamic acid, cysteine or glycine.

In a preferred embodiment, each **A** segment contains 13-15 amino acid residues, including 10-15 alanine residues and 0-3 non-alanine residues as described above. In a more preferred embodiment, each **A** segment contains 13-15 amino acid residues, including 12-15 alanine residues and 0-1 non-alanine residues as described above.

It is preferred that each individual **A** segment has at least 80%, preferably at least 90%, more preferably 95%, most preferably 100% identity to an amino acid sequence selected from the group of amino acid residues 7-19, 43-56, 71-83, 107-120, 135-147, 171-183, 198-211, 235-248, 266-279, 294-306, 330-342, 357-370, 394-406, 421-434, 458-470, 489-502, 517-529, 553-566, 581-594, 618-630, 648-661, 676-688, 712-725, 740-752, 776-789, 804-816, 840-853, 868-880, 904-917, 932-945, 969-981, 999-1013, 1028-1042 and 1060-1073 of SEQ ID NO: 10. Each sequence of this group corresponds to a segment of the naturally occurring sequence of *Euprosthenops australis* MaSp1 protein, which is deduced from cloning of the corresponding cDNA, see WO 2007/078239. Alternatively, each individual **A** segment has at least 80%, preferably at least 90%, more preferably 95%, most preferably 100% identity to an amino acid sequence selected from the group of amino acid residues 143-152, 174-186, 204-218, 233-247 and 265-278 of SEQ ID NO: 3. Each sequence of this group corresponds to a segment of expressed, non-natural spider silk proteins according to the invention, which proteins have capacity to form silk fibers under appropriate conditions (see Example 2). Thus, in certain embodiments according to the invention, each individual **A** segment is identical to an amino acid sequence selected from the above-mentioned amino acid segments. Without wishing to be bound by any particular theory, it is envisaged that **A** segments according to the invention form helical structures or beta sheets.

The term "% identity", as used throughout the specification and the appended claims, is calculated as follows. The query sequence is aligned to the target sequence using the CLUSTAL W algorithm (Thompson, J.D., Higgins, D.G. and Gibson, T.J., Nucleic Acids Research, 22: 4673-4680 (1994)). A comparison is made over the window corresponding to the shortest of the aligned sequences. The amino acid residues at each position are compared, and the percentage of positions in the query sequence that have identical correspondences in the target sequence is reported as % identity.

The term "% similarity", as used throughout the specification and the appended claims, is calculated as described for "% identity", with the exception that the hydrophobic residues Ala, Val, Phe, Pro, Leu, Ile, Trp, Met and Cys are similar; the basic residues Lys, Arg and His are similar; the acidic residues Glu and Asp are similar; and the hydrophilic, uncharged residues Gln, Asn, Ser, Thr and Tyr are similar. The remaining natural amino acid Gly is not similar to any other amino acid in this context.

Throughout this description, alternative embodiments according to the invention fulfill, instead of the specified percentage of identity, the corresponding percentage of similarity. Other alternative embodiments fulfill the specified percentage of identity as well as another, higher percentage of similarity, selected from the group of preferred percentages of identity for each sequence. For example, a sequence may be 70% similar to another sequence; or it may be 70% identical to another sequence; or it may be 70% identical and 90% similar to another sequence.

Furthermore, it has been concluded from experimental data that each individual **G** segment is an amino acid sequence of from 12 to 30 amino acid residues. It is preferred that each individual **G** segment consists of from 14 to 23 amino acid residues. At least 40% of the amino acid residues of each **G** segment are glycine residues. Typically the glycine content of each individual **G** segment is in the range of 40-60%.

It is preferred that each individual **G** segment has at least 80%, preferably at least 90%, more preferably 95%, most preferably 100% identity to an amino acid sequence selected from the group of amino acid residues 20-42, 57-70, 84-106, 121-134, 148-170, 184-197, 212-234, 249-265, 280-293, 307-329, 343-356, 371-393, 407-420, 435-457, 471-488, 503-516, 530-552, 567-580, 595-617, 631-647, 662-675, 689-711, 726-739, 753-775, 790-803, 817-839, 854-867, 881-903, 918-931, 946-968, 982-998, 1014-1027, 1043-1059 and 1074-1092 of SEQ ID NO: 10. Each sequence of this group corresponds to a segment of the naturally occurring sequence of *Euprosthenops australis* MaSp1 protein, which is deduced from cloning of the corresponding cDNA, see WO 2007/078239. Alternatively, each individual **G** segment has at least 80%, preferably at least 90%, more preferably 95%, most preferably 100% identity to an amino acid sequence selected from the group of amino acid residues 153-173, 187-203, 219-232, 248-264 and 279-296 of SEQ ID NO: 3. Each sequence of this group corresponds to a segment of expressed, non-natural spider silk proteins according to the invention, which proteins have capacity to form silk fibers under appropriate conditions (see Example 2). Thus, in certain embodiments according to the invention, each individual **G** segment is identical to an amino acid sequence selected from the above-mentioned amino acid segments.

In certain embodiments, the first two amino acid residues of each **G** segment according to the invention are not -Gln-Gln-.

There are the three subtypes of the **G** segment according to the invention. This classification is based upon careful analysis of the *Euprosthenops australis* MaSp1 protein sequence (WO 2007/078239), and the information has been employed and verified in the construction of novel, non-natural spider silk proteins.

The first subtype of the **G** segment according to the invention is represented by the amino acid one letter consensus sequence GQG(G/S)QGG(Q/Y)GG (L/Q)GQGGYGQGA GSS (SEQ ID NO: 11). This first, and generally the longest, **G** segment subtype typically contains 23 amino acid residues, but may contain as little as 17 amino acid residues, and lacks charged residues or contain one charged residue. Thus, it is preferred that this first **G** segment subtype contains 17-23 amino acid residues, but it is contemplated that it may contain as few as 12 or as many as 30 amino acid residues. Without wishing to be bound by any particular theory, it is envisaged that this subtype forms coil structures or $3_1$-helix structures. Representative **G** segments of this first subtype are amino acid residues 20-42, 84-106, 148-170, 212-234, 307-329, 371-393, 435-457, 530-552, 595-617, 689-711, 753-775, 817-839, 881-903, 946-968, 1043-1059 and 1074-1092 of SEQ ID NO: 10. In certain embodiments, the first two amino acid residues of each **G** segment of this first subtype according to the invention are not -Gln-Gln-.

The second subtype of the **G** segment according to the invention is represented by the amino acid one letter consensus sequence GQGGQGQG(G/R)Y GQG(A/S)G(S/G)S (SEQ ID NO: 12). This second, generally mid-sized, **G** segment subtype typically contains 17 amino acid residues and lacks charged residues or contain one charged residue. It is preferred that this second **G** segment subtype contains 14-20 amino acid residues, but it is contemplated that it may contain as few as 12 or as many as 30 amino acid residues. Without wishing to be bound by any particular theory, it is envisaged that this subtype forms coil structures. Representative **G** segments of this second subtype are amino acid residues 249-265, 471-488, 631-647 and 982-998 of SEQ ID NO: 10; and amino acid residues 187-203 of SEQ ID NO: 3.

The third subtype of the G segment according to the invention is represented by the amino acid one letter consensus sequence G(R/Q)GQG(G/R)YGQG (A/S/V)GGN (SEQ ID NO: 13). This third **G** segment subtype typically contains 14 amino acid residues, and is generally the shortest of the **G** segment subtypes according to the invention. It is preferred that this third **G** segment subtype contains 12-17 amino acid residues, but it is contemplated that it may contain as many as 23 amino acid residues. Without wishing to be bound by any particular theory, it is envisaged that this subtype forms turn structures. Representative G segments of this third subtype are amino acid residues 57-70, 121-134, 184-197, 280-293, 343-356, 407-420, 503-516, 567-580, 662-675, 726-739, 790-803, 854-867, 918-931, 1014-1027 of SEQ ID NO: 10; and amino acid residues 219-232 of SEQ ID NO: 3.

Thus, in preferred embodiments, each individual G segment has at least 80%, preferably 90%, more preferably 95%, identity to an amino acid sequence selected from SEQ ID NO: 11, SEQ ID NO: 12 and SEQ ID NO: 13.

In a preferred embodiment of the alternating sequence of **A** and **G** segments of the **REP** fragment, every second **G** segment is of the first subtype, while the remaining G segments are of the third subtype, e.g. ...$\mathbf{A}_1\mathbf{G}_{short}\mathbf{A}_2\mathbf{G}_{long}\mathbf{A}_3\mathbf{G}_{short}\mathbf{A}_4\mathbf{G}_{long}\mathbf{A}_5\mathbf{G}_{short}$... In another preferred embodiment of the REP fragment, one G segment of the second subtype interrupts the **G** segment regularity *via* an insertion, e.g. ...$\mathbf{A}_1\mathbf{G}_{short}\mathbf{A}_2\mathbf{G}_{long}\mathbf{A}_3\mathbf{G}_{mid}\mathbf{A}_4\mathbf{G}_{short}\mathbf{A}_5\mathbf{G}_{long}$...

Each individual L segment represents an optional linker amino acid sequence, which may contain from 0 to 20 amino acid residues, such as from 0 to 10 amino acid residues. While this segment is optional and not functionally critical for the spider silk protein, its presence still allows for fully functional spider silk proteins, forming spider silk fibers according to the invention. There are also linker amino acid sequences present in the repetitive part (SEQ ID NO: 10) of the deduced amino acid sequence of the MaSp1 protein from *Euprosthenops australis.* In particular, the amino acid sequence of a linker segment may resemble any of the described **A** or **G** segments, but usually not sufficiently to meet their criteria as defined herein.

As shown in WO 2007/078239, a linker segment arranged at the C-terminal part of the **REP** fragment can be represented by the amino acid one letter consensus sequences ASASAAASAA STVANSVS and ASAASAAA, which are rich in alanine. In fact, the second sequence can be considered to be an **A** segment according to the invention, while the first sequence has a high degree of similarity to **A** segments according to the invention. Another example of a linker segment according the invention has the one letter amino acid sequence GSAMGQGS, which is rich in glycine and has a high degree of similarity to **G** segments according to the invention. Another example of a linker segment is SASAG.

Representative **L** segments are amino acid residues 1-6 and 1093-1110 of SEQ ID NO: 10; and amino acid residues 138-142 of SEQ ID NO: 3, but the skilled person in the art will readily recognize that there are many suitable alternative amino acid sequences for these segments. In one embodiment of the **REP** fragment according to the invention, one of the **L** segments contains 0 amino acids, i.e. one of the **L** segments is void. In another embodiment of the **REP** fragment according to the invention, both **L** segments contain 0 amino acids, i.e. both **L** segments are void. Thus, these embodiments of the **REP** fragments according to the invention may be schematically represented as follows: $(\mathbf{AG})_n\mathbf{L}$, $(\mathbf{AG})_n\mathbf{AL}$, $(\mathbf{GA})_n\mathbf{L}$, $(\mathbf{GA})_n\mathbf{GL}$; $\mathbf{L}(\mathbf{AG})_n$, $\mathbf{L}(\mathbf{AG})_n\mathbf{A}$, $\mathbf{L}(\mathbf{GA})_n$, $\mathbf{L}(\mathbf{GA})_n\mathbf{G}$; and $(\mathbf{AG})_n$, $(\mathbf{AG})_n\mathbf{A}$, $(\mathbf{GA})_n$, $(\mathbf{GA})_n\mathbf{G}$. Any of these **REP** fragments are suitable for use with any **CT** fragment as defined below.

The optional **CT** fragment of the spider silk protein according to the invention has a high degree of similarity to the C-terminal amino acid sequence of spider silk proteins. As shown in WO 2007/078239, this amino acid sequence is well conserved among various species and spider silk proteins, including MaSp1 and MaSp2. A consensus sequence of the C-terminal regions of MaSp1 and MaSp2 is provided as SEQ ID NO: 9. In Fig 2, the following MaSp proteins are aligned, denoted with GenBank accession entries where applicable:

TABLE 2 - Spidroin **CT** fragments

| Species and spidroin protein | Entry |
|---|---|
| *Euprosthenops sp* MaSp1 (Pouchkina-Stantcheva, NN & McQueen-Mason, SJ, *ibid*) | Cthyb_Esp |
| *Euprosthenops australis* MaSp1 | CTnat_Eau |
| | |
| *Argiope trifasciata* MaSp1 | AF350266_At1 |
| *Cyrtophora moluccensis* Sp1 | AY666062_Cm1 |
| *Latrodectus geometricus* MaSp1 | AF350273_Lg1 |
| *Latrodectus hesperus* MaSp1 | AY953074_Lh1 |
| *Macrothele holsti* Sp1 | AY666068_Mh1 |
| *Nephila clavipes* MaSp1 | U20329_Nc1 |
| *Nephila pilipes* MaSp1 | AY666076_Np1 |
| *Nephila madagascariensis* MaSp1 | AF350277_Nm1 |
| *Nephila senegalensis* MaSp1 | AF350279_Ns1 |
| *Octonoba varians* Sp1 | AY666057_Ov1 |
| *Psechrus sinensis* Sp1 | AY666064_Ps1 |
| *Tetragnatha kauaiensis* MaSp1 | AF350285_Tk1 |
| *Tetragnatha versicolor* MaSp1 | AF350286_Tv1 |
| | |
| *Araneus bicentenarius* Sp2 | ABU20328_Ab2 |
| *Argiope amoena* MaSp2 | AY365016_Aam2 |
| *Argiope aurantia* MaSp2 | AF350263_Aau2 |
| *Argiope trifasciata* MaSp2 | AF350267_At2 |
| *Gasteracantha mammosa* MaSp2 | AF350272_Gm2 |
| *Latrodectus geometricus* MaSp2 | AF350275_Lg2 |
| *Latrodectus hesperus* MaSp2 | AY953075_Lh2 |
| *Nephila clavipes* MaSp2 | AY654293_Nc2 |
| *Nephila madagascariensis* MaSp2 | AF350278_Nm2 |
| *Nephila senegalensis* MaSp2 | AF350280_Ns2 |
| | |
| *Dolomedes tenebrosus* Fb1 | AF350269_DtFb1 |
| *Dolomedes tenebrosus* Fb2 | AF350270_DtFb2 |
| | |
| *Araneus diadematus* ADF-1 | U47853_ADF1 |
| *Araneus diadematus* ADF-2 | U47854_ADF2 |
| *Araneus diadematus* ADF-3 | U47855_ADF3 |
| *Araneus diadematus* ADF-4 | U47856_ADF4 |

It is not critical which specific **CT** fragment, if any, is present in spider silk proteins according to the invention. Thus, the **CT** fragment according to the invention can be selected from any of the amino acid sequences shown in Fig 2 and Table 2 or sequences with a high degree of similarity. A wide variety of C-terminal sequences can be used in the spider silk protein according to the invention.

The sequence of the **CT** fragment according to the invention has at least 50% identity, preferably at least 60%, more preferably at least 65% identity, or even at least 70% identity, to the consensus amino acid sequence SEQ ID NO: 9, which is based on the amino acid sequences of Fig 2.

A representative **CT** fragment according to the invention is the *Euprosthenops australis* sequence SEQ ID NO: 7, Thus, according to a preferred aspect of the invention, the **CT** fragment has at least 80%, preferably at least 90%, such as at least 95%, identity to SEQ ID NO: 7 or any individual amino acid sequence of Fig 2 and Table 2. In preferred aspects of the invention, the **CT** fragment is identical to SEQ ID NO: 7 or any individual amino acid sequence of Fig 2 and Table 2.

The **CT** fragment typically consists of from 70 to 120 amino acid residues. It is preferred that the **CT** fragment contains at least 70, or more than 80, preferably more than 90, amino acid residues. It is also preferred that the **CT** fragment contains at most 120, or less than 110 amino acid residues. A typical **CT** fragment contains approximately 100 amino acid residues.

In one embodiment, the first step of the method of producing polymers of an isolated spider silk protein involves expression of a polynucleic acid molecule which encodes the spider silk protein in a suitable host, such as *Escherichia coli.* The thus obtained protein is isolated using standard procedures. Optionally, lipopolysaccharides and other pyrogens are actively removed at this stage.

In the second step of the method of producing polymers of an isolated spider silk protein, a solution of the spider silk protein in a liquid medium is provided. By the terms "soluble" and "in solution" is meant that the protein is not visibly aggregated and does not precipitate from the solvent at 60 000×g. The liquid medium can be any suitable medium, such as an aqueous medium, preferably a physiological medium, typically a buffered aqueous medium, such as a 10-50 mM Tris-HCl buffer or phosphate buffer. The liquid medium has a pH of 6.4 or higher and/or an ion composition that prevents polymerisation of the spider silk protein. That is, the liquid medium has either a pH of 6.4 or higher or an ion composition that prevents polymerisation of the spider silk protein, or both.

Ion compositions that prevent polymerisation of the spider silk protein can readily be prepared by the skilled person utilizing the methods disclosed herein. A preferred ion composition that prevents polymerisation of the spider silk protein has an ionic strength of more than 300 mM. Specific examples of ion compositions that prevent polymerisation of the spider silk protein include above 300 mM NaCl, 100 mM phosphate and combinations of these ions having desired preventive effect on the polymerisation of the spider silk protein, e.g. a combination of 10 mM phosphate and 300 mM NaCl.

It has been surprisingly been found that the presence of an **NT** fragment improves the stability of the solution and prevents polymer formation under these conditions. This can be advantageous when immediate polymerisation may be undesirable, e.g. during protein purification, in preparation of large batches, or when other conditions need to be optimized. It is preferred that the pH of the liquid medium is adjusted to 6.7 or higher, such as 7.0 or higher to achieve high solubility of the spider silk protein. It can also be advantageous that the pH of the liquid medium is adjusted to the range of 6.4-6.8, which provides sufficient solubility of the spider silk protein but facilitates subsequent pH adjustment to 6.3 or lower.

In the third step, the properties of the liquid medium are adjusted to a pH of 6.3 or lower and ion composition that allows polymerisation. That is, if the liquid medium wherein the spider silk protein is dissolved has a pH of 6.4 or higher, the pH is decreased to 6.3 or lower. The skilled person is well aware of various ways of achieving this, typically involving addition of a strong or weak acid. If the liquid medium wherein the spider silk protein is dissolved has an ion composition that prevents polymerisation, the ion composition is changed so as to allow polymerisation. The skilled person is well aware of various ways of achieving this, e.g. dilution, dialysis or gel filtration. If required, this step involves both decreasing the pH of the liquid medium to 6.3 or lower and changing the ion composition so as to allow polymerisation. It is preferred that the pH of the liquid medium is adjusted to 6.2 or lower, such as 6.0 or lower. In particular, it may be advantageous from a practical point of view to limit the pH drop from 6.4 or 6.4-6.8 in the preceding step to 6.3 or 6.0-6.3, e.g. 6.2 in this step.

In the fourth step, the spider silk protein is allowed to polymerise in the liquid medium having pH of 6.3 or lower and an ion composition that allows polymerisation of the spider silk protein. It has surprisingly been found that although the presence of the **NT** fragment improves solubility of the spider silk protein at a pH of 6.4 or higher and/or an ion composition that prevents polymerisation of the spider silk protein, it accelerates polymer formation at a pH of 6.3 or lower when the ion composition allows polymerisation of the spider silk protein. The resulting polymers are solid and macroscopic, and they are formed in the liquid medium having a pH of 6.3 or lower and an ion composition that allows polymerisation of the spider silk protein. Preferred polymer shapes include a fiber, film, foam, net or mesh. It is preferred that the polymer is a fiber having a diameter of more than 0.1 μm, preferably more than 1 μm, and a length of more than 5 mm.

Ion compositions that allow polymerisation of the spider silk protein can readily be prepared by the skilled person utilizing the methods disclosed herein. A preferred ion composition that allows polymerisation of the spider silk protein has an ionic strength of less than 300 mM. Specific examples of ion compositions that allow polymerisation of the spider silk protein include 150 mM NaCl, 10 mM phosphate and combinations of these ions lacking preventive effect on the polymerisation of the spider silk protein, e.g. a combination of 10 mM phosphate and 150 mM NaCl. It is preferred that the ionic strength of this liquid medium is adjusted to the range of 1-250 mM.

Without desiring to be limited to any specific theory, it is envisaged that the **NT** fragments have oppositely charged poles, and that environmental changes in pH affects the charge balance on the surface of the protein followed by polymerisation, whereas salt inhibits the same event.

At neutral pH, the energetic cost of burying the excess negative charge of the acidic pole may be expected to prevent polymerisation. However, as the dimer approaches its isolectric point at lower pH, attractive electrostatic forces will eventually become dominant, explaining the observed salt and pH-dependent polymerisation behaviour of **NT** and **NT**-containing minispidroins. We propose that pH-induced **NT** polymerisation, and increased efficiency of fiber assembly of **NT**-minispidroins, are due to surface electrostatic potential changes, and that clustering of acidic residues at one pole of **NT** shifts its charge balance such that the polymerisation transition occurs at pH values of 6.3 or lower.

In the fifth and final step, the resulting solid spider silk protein polymers are isolated from said liquid medium. Optionally, this step involves actively removing lipopolysaccharides and other pyrogens from the spidroin polymers.

Without desiring to be limited to any specific theory, it has been observed that formation of spidroin polymers progresses via formation of water-soluble spidroin dimers. The present invention thus also provides a method of producing dimers of an isolated spider silk protein, wherein the first two method steps are as described above. The spider silk protein are present as dimers in a liquid medium at a pH of 6.4 or higher and/or an ion composition that prevents polymerisation of said spider silk protein. The third step involves isolating the dimers obtained in the second step, and optionally removal of lipopolysaccharides and other pyrogens. In a preferred embodiment, the spider silk protein polymer of the invention consists of polymerised protein dimers. The present invention thus provides a novel use of a spider silk protein, preferably those disclosed herein, for producing dimers of the spider silk protein.

According to another aspect, the present invention provides a polymer of a spider silk protein as disclosed herein. In a preferred embodiment, the polymer of this protein is obtainable by any one of the methods therefor according to the invention. Thus, the present invention provides a novel use of a spider silk protein, preferably those disclosed herein, for producing polymers of the spider silk protein. According to one embodiment, the present invention provides a novel use of a dimer of a spider silk protein, preferably those disclosed herein, for producing polymers of the isolated spider silk protein. In these uses, it is preferred that the polymers are produced in a liquid medium having a pH of 6.3 or lower and an ion composition that allows polymerisation of said spider silk protein.

Using the method(s) of the present invention, it is possible to control the polymerization process, and this allows for optimization of parameters for obtaining silk polymers with desirable properties and shapes.

It is preferable that the polymer of the spidroin protein according to the invention is a fiber with a macroscopic size, i.e. with a diameter above 0.1 μm, preferably above 1 μm, and a length above 5 mm. It is preferred that the fiber has a diameter in the range of 1-400 μm, preferably 60-120 μm, and a length in the range of 0.5-300 cm, preferably 1-100 cm. Other preferred ranges are 0.5-30 cm and 1-20 cm. It is also preferred that the polymer of the spidroin protein according to the invention has a tensile strength above 1 MPa, preferably above 2 MPa, more preferably 10 MPa or higher. It is preferred that the polymer of the spidroin protein according to the invention has a tensile strength above 100 MPa, more preferably 200 MPa or higher. The fiber has the capacity to remain intact during physical manipulation, i.e. can be used for spinning, weaving, twisting, crocheting and similar procedures.

In other preferred embodiments, the polymer of the spidroin protein according to the invention forms a foam, a net, a mesh or a film.

According to another aspect, the present invention provides an isolated polynucleic acid molecule comprising a nucleic acid sequence which encodes a spider silk protein according to the invention, or its complementary nucleic acid sequence, such as SEQ ID NO: 14-16. These polynucleic acid molecules as well as polynucleic acid molecules coding for the various proteins disclosed herein (SEQ ID NO: 1-7, 10-13) may also be useful in further developments of non-natural spidroin proteins or production systems therefor.

Polynucleic acid molecules according to the invention can be DNA molecules, including cDNA molecules, or RNA molecules. As the skilled person is well aware, a nucleic acid sequence may as well be described by its complementary nucleic acid sequence. Therefore, nucleic acid sequences that are complementary to the nucleic acid sequences according to the invention are also encompassed by the protective scope of the invention.

According to one aspect, the present invention provides a method of producing a spider silk protein according to the invention. In the first step, a polynucleic acid molecule which encodes a spider silk protein according to the invention is expressed in a suitable host. In the second step, the thus obtained soluble spider silk protein is isolated, e.g. using chromatography and/or filtration. Optionally, said second step of isolating the soluble spider silk protein involves removal of LPS and other pyrogens.

The spider silk protein according to the invention is typically recombinantly produced using a variety of suitable hosts, such as bacteria, yeast, mammalian cells, plants, insect cells, and transgenic animals. It is preferred that the spider silk protein according to the invention is produced in bacteria.

In order to obtain a protein with low pyrogenic content, which is an obligate for usage as a biomaterial *in vivo,* a purification protocol optimized for removal of lipopolysaccharides (LPS) has been developed. To avoid contamination by released LPS, the producing bacterial cells are subjected to washing steps with altering $CaCl_2$ and EDTA. After cell lysis, all subsequent purifications steps are performed in low conductivity buffers in order to minimize hydrophobic interactions between the target protein and LPS. The LPS content is further minimized by passage of the protein solution through an Endotrap column, which has a ligand that specifically adsorbs LPS. To assure constant low content of LPS and other pyrogens, all batches are analyzed using an *in vitro* pyrogen test (IPT) and/or a Limulus amebocyte lysate (LAL) kinetic assay. Although produced in a gram-negative bacterial host, the recombinant spidroin proteins can be purified so that residual levels of LPS and other pyrogens are below the limits required for animal tests, i.e. below 25 EU/implant. In certain embodiments according to the invention, the content of LPS and other pyrogens in the isolated spider silk protein is 1 EU/mg protein or lower. In certain embodiments according to the invention, the content of LPS and other pyrogens in the isolated spider silk protein is 1 EU/mg protein or lower, preferably 0.25 EU/mg protein or lower.

According to one aspect, the present invention provides a composition comprising an isolated spider silk protein, preferably those disclosed herein, dissolved in a liquid medium having a pH of 6.4 or higher and/or an ion composition that prevents polymerisation of said spider silk protein. The liquid medium can be any suitable medium, such as an aqueous medium, preferably a physiological medium, typically a buffered aqueous medium, such as a 10-50 mM Tris-HCl buffer or phosphate buffer. The liquid medium has a pH of 6.4 or higher and/or an ion composition that prevents polymerisation of the spider silk protein. That is, the liquid medium has either a pH of 6.4 or higher or an ion composition that prevents polymerisation of the spider silk protein, or both. A preferred ion composition that prevents polymerisation of the spider silk protein has an ionic strength of more than 300 mM. Specific examples of ion compositions that prevent polymerisation of the spider silk protein include above 300 mM NaCl, 100 mM phosphate and combinations of these ions having desired preventive effect on the polymerisation of the spider silk protein, e.g. a combination of 10 mM phosphate and 300 mM NaCl. It is preferred that the pH of the liquid medium is 6.7 or higher, such as 7.0 or higher to achieve high solubility of the spider silk protein. It can also be advantageous that the pH of the liquid medium is in the range of 6.4-6.8, which provides sufficient solubility of the spider silk protein but facilitates subsequent pH adjustment to 6.3 or lower. It is preferred that the content of lipopolysaccharides and other pyrogens is 1 EU/mg of isolated protein or lower in the liquid medium.

The inventive insights that the N-terminal non-repetitive fragment of spider silk proteins is involved in polymerisation of these proteins and that the formation of polymers involving this fragment can be tightly controlled by varying certain parameters have also been developed into a novel method of reversibly assembling a polymer or oligomer of molecules carrying the N-terminal non-repetitive spidroin fragment. Although the examples by necessity relate to specific proteins, in this case containing N-terminal protein fragments derived from major spidroin 1 (MaSp1) from *Euprosthenops australis,* it is considered that the method disclosed herein is applicable to any similar protein for the purpose of producing polymers or oligomers.

According to this aspect, the present invention provides a method of reversibly assembling a polymer or oligomer of one type of molecule or several different types of molecules. The first method step involves providing said molecules. Each molecule is comprising (a) a first binding moiety and (b) a second moiety that is carrying a bioactivity to be studied or utilized. Each binding moiety (a) consists of from of from 100 to 160 amino acid residues, and it is derived from the N-terminal (**NT**) fragment of a spider silk protein. The **NT** fragment has a high degree of similarity to the N-terminal amino acid sequence of spider silk proteins. As shown in Table 1 and Fig 1, this amino acid sequence is well conserved among various species and spider silk proteins, including MaSp1 and MaSp2.

It is observed that **NT** has a clear dipole moment as acidic and basic residues are localized in clusters at opposite poles. Without desiring to be limited thereto, it is contemplated that the observed polymerisation of **NT** may involve the formation of linear arrays of **NT** dimers, stacked pole-to-pole with the negative surface of one subunit facing the positive surface of the neighbouring subunit in the next dimer in the array.

It is not critical which specific **NT** fragment is present in the molecule type(s) according to this aspect of the invention, as long as the **NT** fragment is not entirely missing. Thus, the **NT** fragment according to this aspect of the invention can be selected from any of the amino acid sequences shown in Table 1 or Fig 1 or sequences with a high degree of similarity. A wide variety of N-terminal sequences can be used in the molecule type(s) according to this aspect of the invention.

The sequence of the **NT** fragment according to the invention has at least 50% identity, preferably at least 60% identity, to the consensus amino acid sequence SEQ ID NO: 8, which is based on the amino acid sequences of Fig 1. In a preferred embodiment, the sequence of the **NT** fragment according to the invention has at least 65% identity, preferably at least 70% identity, to the consensus amino acid sequence SEQ ID NO: 8. In preferred embodiments, the **NT** fragment according to the invention has furthermore 70%, preferably 80%, similarity to the consensus amino acid sequence SEQ ID NO: 8.

A representative **NT** fragment according to the invention is the *Euprosthenops australis* sequence SEQ ID NO 6: According to a preferred embodiment of the invention, the **NT** fragment has at least 80% identity to SEQ ID NO: 6 or any individual amino acid sequence in Fig 1. In preferred embodiments of the invention, the **NT** fragment has at least 90%, such as at least 95% identity, to SEQ ID NO: 6 or any individual amino acid sequence in Fig 1. In preferred embodiments of the invention, the **NT** fragment is identical to SEQ ID NO: 6 or any individual amino acid sequence in Fig 1.

The **NT** fragment contains from 100 to 160 amino acid residues. It is preferred that the **NT** fragment contains at least 100, or more than 110, preferably more than 120, amino acid residues. It is also preferred that the **NT** fragment contains at most 160, or less than 140 amino acid residues. A typical **NT** fragment contains approximately 130-140 amino acid residues.

All molecules of a particular method typically have the binding moiety (a) in common, but it is also possible to have different molecule types where the difference resides in use of different binding moieties (a). In general, the second moiety (b) is carrying a bioactivity to be studied or utilized, and it is typically this second moiety (b) that differs when the method involves more than one molecule type. The second moiety (b) is individually selected from proteins, nucleic acids, carbohydrates and lipids. Preferably, the second moiety (b) is also a protein.

In a preferred embodiment, the molecules of the first step are identical, i.e. of a single type, and the resulting polymer (oligomer) is thus a homopolymer (homooligomer). In another preferred embodiment, the molecules of the first step are not identical, and the resulting polymer (oligomer) is thus a heteropolymer (heterooligomer). As discussed above, the molecule heterogeneity may reside in the binding moiety (a), the bioactivity moiety (b), or both.

In the second method step, a solution of the molecules in a liquid medium is provided. The liquid medium can be any suitable medium, such as an aqueous medium, preferably a physiological medium, typically a buffered aqueous medium, such as a 10-50 mM Tris-HCl buffer or phosphate buffer. The liquid medium has a pH of 6.4 or higher and/or an ion composition that prevents polymerisation or oligomerisation of the molecules *via* the binding moieties. That is, the liquid medium has either a pH of 6.4 or higher or an ion composition that prevents polymerisation or oligomerisation of the molecules *via* the binding moieties, or both.

Ion compositions that prevent polymerisation or oligomerisation of the molecules *via* the binding moieties can readily be prepared by the skilled person utilizing the methods disclosed herein. A preferred ion composition that prevents polymerisation of the molecules *via* the binding moieties has an ionic strength of more than 300 mM. Specific examples of ion compositions that prevent polymerisation of the molecules *via* the binding moieties include above 300 mM NaCl, 100 mM phosphate and combinations of these ions having desired preventive effect on the polymerisation of the molecules *via* the binding moieties, e.g. a combination of 10 mM phosphate and 300 mM NaCl.

It has been surprisingly been found that the presence of an NT fragment improves the stability of the solution and prevents polymer and oligomer formation under these conditions. This can be advantageous when immediate polymerisation or oligomerisation may be undesirable, e.g. during protein purification, in preparation of large batches, or when other conditions need to be optimized. It is preferred that the pH of the liquid medium is adjusted to 6.7 or higher, such as 7.0 or higher to achieve high solubility of the molecules. It can also be advantageous that the pH of the liquid medium is adjusted to the range of 6.4-6.8, which provides sufficient solubility of the molecules but facilitates subsequent pH adjustment to 6.3 or lower.

In the third method step, the properties of said liquid medium are adjusted so as to allow polymerisation or oligomerisation of the molecules *via* the binding moieties. The properties of the liquid medium are therefore adjusted to a pH of 6.3 or lower and ion composition that allows polymerisation or oligomerisation. That is, if the liquid medium wherein the molecules is dissolved has a pH of 6.4 or higher, the pH is decreased to 6.3 or lower. The skilled person is well aware of various ways of achieving this, typically involving addition of a strong or weak acid. If the liquid medium wherein the molecules are dissolved has an ion composition that prevents polymerisation or oligomerisation, the ion composition is changed so as to allow polymerisation or oligomerisation of the molecules *via* the binding moieties. The skilled person is well aware of various ways of achieving this, e.g. dilution, dialysis or gel filtration. If required, this step involves both decreasing the pH of the liquid medium to 6.3 or lower and changing the ion composition so as to allow polymerisation or oligomerisation. It is preferred that the pH of the liquid medium is adjusted to 6.2 or lower, such as 6.0 or lower. In particular, it may be advantageous from a practical point of view to limit the pH drop from 6.4 or 6.4-6.8 in the preceding step to 6.3 or 6.0-6.3, e.g. 6.2 in this step.

In the fourth method step, the molecules are allowed to assemble into a polymer or oligomer *via* the binding moieties in the liquid medium. The liquid medium has a pH of 6.3 or lower and an ion composition that allows polymerisation or oligomerisation of the molecules via the binding moieties. It has surprisingly been found that although the presence of the binding moiety improves solubility of the molecules at a pH of 6.4 or higher and/or an ion composition that prevents polymerisation or oligomerisation of the molecules, it accelerates polymer and oligomer formation at a pH of 6.3 or lower when the ion composition allows polymerisation or oligomerisation of the molecules. In a preferred embodiment of this method, the polymer or oligomer that is obtained in the fourth step remains soluble, i.e. it is dissolved in a liquid medium having a pH of 6.3 or lower and an ion composition that allows polymerisation or oligomerisation of the molecules.

Ion compositions that allow polymerisation or oligomerisation of the molecules *via* the binding moieties can readily be prepared by the skilled person utilizing the methods disclosed herein. A preferred ion composition that allows polymerisation of the molecules *via* the binding moieties has an ionic strength of less than 300 mM. Specific examples of ion compositions that allow polymerisation of the molecules *via* the binding moieties include 150 mM NaCl, 10 mM phosphate and combinations of these ions lacking preventive effect on the polymerisation of the molecules *via* the binding moieties, e.g. a combination of 10 mM phosphate and 150 mM NaCl. It is preferred that the ionic strength of this liquid medium is adjusted to the range of 1-250 mM.

In a preferred embodiment, the method according to this aspect of the invention can comprise a fifth step of reversing the polymer or oligomer assembly. This method step involves adjusting the properties of the liquid medium to a pH of 6.4 or higher and/or an ion composition that prevents polymerisation or oligomerisation of said molecules. This causes the polymer or oligomer that is present in the liquid medium to disassemble and dissolve in the liquid medium.

The liquid medium of this fifth method step can have the same composition as discussed for the liquid medium of the second method step. For instance, it is preferred that the pH of the liquid medium of the fifth method step is 6.7 or higher, such as 7.0 or higher. Alternatively, the pH of the liquid medium of the fifth method step is in the range of 6.4-6.8.

The polymer or oligomer of step (iv) can advantageously be used in interaction studies, separation, inducing activity of co-enzyme complexes or FRET analysis. In certain applications, at least one molecule type of the first method step is immobilised to a solid support.

According to one aspect, the present invention also provides method of detecting binding interactions between a subset of molecules comprised in a set of molecules. In the first method step, a set of molecules is provided. Each molecule of this set is designed as detailed above, i.e. it is comprising (a) a first binding moiety and (b) a second moiety that is carrying a bioactivity to be studied or utilized. Each binding moiety (a) consists of from of from 100 to 160 amino acid residues, and it is derived from the N-terminal (NT) fragment of a spider silk protein as set out above. Each bioactivity moiety (b) is individually selected from proteins, nucleic acids, carbohydrates and lipids, preferably proteins.

In the second method step, a solution of said set of molecules in a liquid medium is provided. As set out above, the liquid medium has at pH 6.4 or higher and/or an ion composition that prevents polymerisation or oligomerisation of said molecules. Preferred compositions of the liquid medium are evident from the previous disclosure.

In the third method step, the properties of the liquid medium are adjusted to allow polymerisation or oligomerisation of the molecules. As set out above, the liquid medium has a pH of 6.3 or lower and an ion composition that allows polymerisation or oligomerisation of the molecules. Preferred compositions of the liquid medium are evident from the previous disclosure.

In the fourth method step, the molecules of this set are allowed to assemble into a polymer or oligomer via said binding moieties in the liquid medium. As set out above, the liquid medium has a pH of 6.3 or lower and an ion composition that allows polymerisation or oligomerisation of the molecules. Preferred compositions of the liquid medium are evident from the previous disclosure.

In the fifth method step, the properties of the liquid medium are adjusted so as to disassemble the polymer or oligomer. As set out above, the liquid medium has at pH 6.4 or higher and/or an ion composition that prevents polymerisation or oligomerisation of said molecules. Preferred compositions of the liquid medium are evident from the previous disclosure. This causes disassembly of the polymer or oligomer by preventing association via the NT-derived binding moieties.

In the final and sixth method step, the presence of binding interactions which are not mediated via said binding moieties between two or more different molecules are determined. This identifies binding interactions between a subset of molecules that do not involve the pH/salt-regulated polymerisation or oligomerisation that is mediated via the NT-derived binding moieties.

According to a related aspect, the present invention provides a novel use of one or more molecules. As set out above, each molecule is comprising (a) a first binding moiety of from 100 to 160 amino acid residues which is derived from the N-terminal fragment of a spider silk protein, and (b) a second moiety which is individually selected from proteins, nucleic acids, carbohydrates and lipids. The molecules are used for reversibly assembling a polymer or oligomer of the molecules *via* the binding moieties in a solution at a pH of 6.3 or lower and an ion composition that allows polymerisation or oligomerisation of said molecules. Preferably, the resulting polymer or oligomer is used in interaction studies, separation, inducing activity of co-enzyme complexes or FRET analysis.

The results and conclusions disclosed herein provide new insights in spider silk assembly at the molecular level. Without desiring to be limited to any particular theory, the polar and unbalanced charge distribution of NT is ideally suited for generation of a polymerisable module that can be simply controlled by pH and salt concentration. This in turn allows NT to regulate silk assembly by preventing premature aggregation and triggering polymerisation as the pH is lowered, similar to what is perceived to occur along the spider's silk extrusion duct.

The present invention will in the following be further illustrated by the following non-limiting examples.

## Materials and Methods

### *Protein expression and purification*

Expression vectors were constructed to produce NT (SEQ ID NO: 6), NTΔHis6, NT5Rep (SEQ ID NO: 4), NT4Rep (SEQ ID NO: 3), and 4RepCT (SEQ ID NO: 2), respectively, as C-terminal fusions to $His_6TrxHis_6$, and NT4RepCT (SEQ ID NO: 5)as an N-terminal fusion to $His_6$. The different vectors were used to transform *Escherichia coli* BL21 (DE3) cells (Merck Biosciences) that were grown at 30°C in Luria-Bertani medium containing kanamycin to an OD600 of ~1, induced with isopropyl-β-D-thiogalactopyranoside, and further incubated for up to 4 hours at room temperature. Lysis, immobilised metal affinity purification and proteolytic removal of the $His_6TrxHis_6$-tag was performed as described in Hedhammar, M. et al. Biochemistry 47, 3407-3417, (2008).

*Dynamic light scattering (DLS)*

The effect of pH and ionic strength on the hydrodynamic diameter of NT and NTΔHis6 (to exclude that pH dependent effects are caused by His at position 6) was measured at 25 ± 0.1 °C in a Zetasizer Nano S from Malvern Instruments (Worcestershire, UK) equipped with a 633 nm HeNe laser. The buffers were filtered through nylon filters prior to use. The sample volume was 50 μl and ZEN2112 low glass cuvettes were used. The attenuation and measurement positions from the cuvette wall (4.65 mm) were kept constant for all analyses. Six scans were performed for each sample. All samples were analyzed in triplicate. The hydrodynamic diameter (dH) was calculated using the General Purpose algorithm in the Malvern software for DLS analysis, which correlates the diffusion coefficient to the hydrodynamic diameter through the Stokes-Einstein equation:

$$d_H = \frac{k_B T}{3\pi\eta D}$$

where $k_B$ is the Boltzmann constant, T is the temperature, η is the viscosity and D is the translational diffusion coefficient. The viscosity and refractive index values of the solvent were obtained from the Malvern software. The Multiple Narrow Modes algorithm was used to verify the results obtained by the General Purpose method. NT and NTΔHis6 samples were analyzed at a concentration of 0.8 mg/ml.

*Turbidimetry*

Turbidity was estimated from the apparent absorbance at 340 nm of proteins (0.8 mg/ml) at different pH values at 25°C in an SLM 4800S spectrofluorimeter equipped with OLIS electronics and software (OLIS Inc. Bogart, GA). NT, NT4Rep, and NTΔHis6 were analysed, with essentially the same results.

*Fiber formation and scanning electron microscopy (SEM)*

Conditions for fiber formation were essentially as described in Stark, M. et al. Biomacromolecules 8, 1695-1701, (2007). Approximately 25 μM of each protein was incubated in 20 mM Na phosphate buffer at pH 7 or 6, with or without 300 mM NaCl. At different time points, samples were applied on SEM stubs, where they were air-dried and vacuum-coated with gold and palladium. The samples were photographed with a LEO 1550 FEG microscope (Carl Zeiss, Oberkochen, Germany) using an acceleration voltage of 5 kV.

<u>Examples</u>

<u>Example 1 - Expression and purification of NT and minispirdoins</u>

Expression vectors (SEQ ID NO: 14-16 and others) were constructed to produce NT (SEQ ID NO: 6), NTΔHis6, NT5Rep (SEQ ID NO: 4), NT4Rep (SEQ ID NO: 3), and 4RepCT (SEQ ID NO: 2), respectively, as C-terminal fusions to $His_6TrxHis_6$, and NT4RepCT (SEQ ID NO: 5) as an N-terminal fusion to $His_6$. The different vectors were used to transform *Escherichia coli* BL21 (DE3) cells (Merck Biosciences) that were grown at 30°C in Luria-Bertani medium containing kanamycin to an OD600 of ~1, induced with isopropyl-β-D-thiogalactopyranoside, and further incubated for up to 4 hours at room temperature. Thereafter, cells were harvested and resuspended in 20 mM Tris-HCl (pH 8.0) supplemented with lysozyme and DNase I. After complete lysis, the 15000g supernatants were loaded onto a column packed with Ni- NTA Sepharose (GE Healthcare, Uppsala, Sweden). The column was washed extensively before bound proteins were eluted with 300 mM imidazole. Fractions containing the target proteins were pooled and dialyzed against 20 mM Tris-HCl (pH 8.0). MaSp1 proteins were released from the tags by proteolytic cleavage using a thrombin:fusion protein ratio of 1:1000 (w/w) at room temperature for 1-2 h. To remove the released HisTrxHis tag, the cleavage mixture was loaded onto a second Ni-NTA Sepharose column and the flowthrough was collected. Protein samples were separated via SDS-PAGE and then stained with Coomassie Brilliant Blue R-250. The proteins were concentrated by ultrafiltration using a 5 kDa molecular mass cutoff cellulose filter (Millipore).

<u>Example 2 - pH-dependent polymerisation of NT and minispidroins</u>

Polymerisation of mini-spidroins with (NT4RepCT or NT4Rep) or without NT (4RepCT or 4Rep) was performed at pH 7 (Fig 3, above time scale) or at pH 6 (Fig 3, below time scale).

Miniature spidroins consisting of repeat regions, with or without the C-terminal domain show no sensitivity towards environmental changes, such as pH fluctuations (4Rep and 4RepCT; Fig. 3). To test the hypothesis that it is the N-terminal domain that is responsible for pH-dependent spidroin polymerisation, several constructs encompassing the N-terminal domain of major ampullate spidroin (MaSp) 1 from *Euprosthenops australis* (NT, NT4Rep and NT4RepCT; Fig. 3) were used to obtain purified recombinant proteins (Example 1). Dynamic light scattering, turbidimetry, and scanning electron microscopy were used to probe the effect of pH and salt concentration on solubility and polymerisation of NT alone, as well as of the minispidroin constructs.

NT and NT4Rep were subjected to turbidimetry at different pH values. Mean values (±SD, n=3) of NT4Rep (circles) and NT (squares) are shown in Fig 4. Similar results were obtained for NT5Rep.

NT was subjected to dynamic light scattering at pH 6-7 and 0-300 mM NaCl. Representative examples of three experiments is shown in Fig 5. See also Table 3

TABLE 3 - Size of the protein determined by dynamic light scattering

| Sample | Size (nm) | % particles |
|---|---|---|
| 100 mM phosphate buffer, pH 7.2 | 4.2 ±0.1 | 99.9% |
| 100 mM phosphate buffer, pH 6.2 | 4.2 ±0.1 | 99.9% |
| 10 mM phosphate buffer + 150 mM NaCl, pH 7.2 | 4.1 ±0.1 | 99.9% |
| 10 mM phosphate buffer + 150 mM NaCl, pH 6.1 | 710 ±142 | 96.8% |
| 10 mM phosphate buffer, pH 7.2 | 4.5 ±0.1 | 99.8% |
| 10 mM phosphate buffer, pH 6.0 | 687 ±50 | 100% |
| 10 mM phosphate buffer + 300 mM NaCl, pH 7.2 | 4.3 ±0.3 | 99.9% |
| 10 mM phosphate buffer + 300 mM NaCl, pH 6.2 | 4.4 ±0.3 | 99.9% |

Alone, NT forms a remarkably soluble (>210 mg/ml) dimer at pH 7.0, but instantly forms polymers with a hydrodynamic size of ~700 nm below pH 6.4 (Fig 4 and 5). NT polymerisation is easily reversed by an increase of pH and blocked by high levels of salt (Fig 5 and 6). These properties are maintained in NTΔHis6 (turbidimetry) and are propagated into minispidroins that include the NT domain (NT4RepCT or NT4Rep), which thereby gain solubility at pH 7 but quickly polymerise at pH 6 (Fig 3).

The arrows in Fig 3 indicate when macroscopic formations first were detected, showing that at pH 7 the presence of NT delays polymerisation, while at pH 6 it accelerates polymerisation. This is independent of whether the C-terminal domain (filled circle) is present or not (indicated by the striped circle). Moreover, the presence of NT results in more ordered polymerisation, exemplified by the scanning electron micrographs in Fig 3, which are representative early polymers for all constructs at pH 7 (above time scale) or for NT4RepCT at pH 6 (below time scale). The observed effects of pH and salt suggest that spidroin polymerisation depends on electrostatic interactions involving NT.

Example 3 - NT as a mediator of pH-dependent and reversible interactions

The N-terminal domain (NT) of major ampullate spidroin 1 from the dragline of Euprosthenops australis is highly soluble (>210 mg/ml) at pH 7 but polymerises via charge interactions into ~700 nm polymers at pH values below 6.4 (shown by dynamic light scattering and turbidimetry). The NT polymerisation is easily reversed by an increase of pH and blocked by high levels of salt. These polymerisation properties are propagated into fusion proteins that include the NT domain (e.g. NT-X and NT-Y), which thereby gain solubility at pH 7 but quickly polymerise at pH 6 (Fig. 7). This reversible way of assembling two different proteins can be used in studies of interactions between proteins, nucleic acids, carbohydrates or lipids, for example analyses of protein-protein interactions employing fluorescence resonance energy transfer, or in induction of activities, for example enzyme activities, or for localization or immobilization of proteins, nucleic acids, carbohydrates or lipids, or in analysis or separation of proteins, nucleic acids, carbohydrates or lipids, for example using array techniques.

## Claims

1. A method of producing polymers of an isolated spider silk protein, comprising the steps of:

(i) providing a spider silk protein consisting of from 170 to 600 amino acid residues and comprising:

an N-terminal fragment of from 100 to 160 amino acid residues derived from the N-terminal fragment of a spider silk protein; and
a repetitive fragment of from 70 to 300 amino acid residues derived from the repetitive fragment of a spider silk protein; and optionally
a C-terminal fragment of from 70 to 120 amino acid residues, which fragment is derived from the C-terminal fragment of a spider silk protein;

(ii) providing a solution of said spider silk protein in a liquid medium at pH 6.4 or higher and/or an ion composition that prevents polymerisation of said spider silk protein, optionally involving removal of lipopolysaccharides and other pyrogens;
(iii) adjusting the properties of said liquid medium to a pH of 6.3 or lower and an ion composition that allows polymerisation of said spider silk protein;
(iv) allowing the spider silk protein to form solid polymers in the liquid medium, said liquid medium having a pH of 6.3 or lower and an ion composition that allows polymerisation of said spider silk protein; and
(v) isolating the solid spider silk protein polymers from said liquid medium.

**2.** A method according to claim 1, wherein the pH of the liquid medium of steps (iii) and (iv) is 6.2 or lower, such as 6.0 or lower.

**3.** A method according to any one of claims 1-2, wherein the ionic strength of the liquid medium of steps (iii) and (iv) is in the range of 1-250 mM.

**4.** A method according to any one of claims 1-3, wherein the pH of the liquid medium of step (ii) is 6.7 or higher, such as 7.0 or higher.

**5.** A method according to any one of claims 1-4, wherein said polymer is a fiber, film, foam, net or mesh.

**6.** A method according to claim 5, wherein said polymer is a fiber having a diameter of more than 0.1 μm and a length of more than 5 mm.

**7.** A method according to any one of claims 1-6, wherein said protein is selected from the group of proteins defined by the formulas **NT-REP-CT** and **NT-REP,** wherein
**NT** is a protein fragment having from 100 to 160 amino acid residues, which fragment is a N-terminal fragment derived from a spider silk protein.
**REP** is a protein fragment having from 70 to 300 amino acid residues, wherein said fragment is selected from the group of **$L(AG)_nL$, $L(AG)_nAL$, $L(GA)_nL$, $L(GA)_nGL$,** wherein
n is an integer from 2 to 8;
each individual **A** segment is an amino acid sequence of from 8 to 18 amino acid residues, wherein from 0 to 3 of the amino acid residues are not Ala, and the remaining amino acid residues are Ala;
each individual **G** segment is an amino acid sequence of from 12 to 30 amino acid residues, wherein at least 40% of the amino acid residues are Gly; and each individual **L** segment is a linker amino acid sequence of from 0 to 20 amino acid residues; and
**CT** is a protein fragment having from 70 to 120 amino acid residues, which fragment is a C-terminal fragment derived from a spider silk protein.

**8.** A polymer of a spider silk protein, said protein consisting of from 170 to 600 amino acid residues and comprising:

an N-terminal fragment of from 100 to 160 amino acid residues derived from the N-terminal fragment of a spider silk protein; and
a repetitive fragment of from 70 to 300 amino acid residues derived from the repetitive fragment of a spider silk protein; and optionally
a C-terminal fragment of from 70 to 120 amino acid residues, which fragment is derived from the C-terminal fragment of a spider silk protein.

**9.** A polymer of a spider silk protein according to claim 8, wherein said protein is selected from the group of proteins defined by the formulas **NT-REP-CT** and **NT-REP,** wherein
**NT** is a protein fragment having from 100 to 160 amino acid residues, which fragment is a N-terminal fragment derived from a spider silk protein.
**REP** is a protein fragment having from 70 to 300 amino acid residues, wherein said fragment is selected from the group of **$L(AG)_nL$, $L(AG)_nAL$, $L(GA)_nL$, $L(GA)_nGL$,** wherein
n is an integer from 2 to 8;
each individual **A** segment is an amino acid sequence of from 8 to 18 amino acid residues, wherein from 0 to 3 of the amino acid residues are not Ala, and the remaining amino acid residues are Ala;
each individual **G** segment is an amino acid sequence of from 12 to 30 amino acid residues, wherein at least 40% of the amino acid residues are Gly; and each individual **L** segment is a linker amino acid sequence of from 0 to 20 amino acid residues; and
**CT** is a protein fragment having from 70 to 120 amino acid residues, which fragment is a C-terminal fragment derived from a spider silk protein.

**10.** A polymer of a spider silk protein according to any one of claims 8-9, wherein said polymer is a fiber, film, foam, net or mesh.

**11.** A polymer of a spider silk protein according to claim 10, wherein said polymer is a fiber having a diameter of more than 0.1 μm and a length of more than 5 mm.

**12.** An isolated spider silk protein, which consists of from 170 to 600 amino acid residues and is selected from the group of proteins defined by the formulas **NT-REP-CT** and **NT-REP,** wherein
**NT** is a protein fragment having from 100 to 160 amino acid residues, which fragment is a N-terminal fragment derived from a spider silk protein.
**REP** is a protein fragment having from 70 to 300 amino acid residues, wherein said fragment is selected from the group of $\mathbf{L(AG)_nL}$, $\mathbf{L(AG)_nAL}$, $\mathbf{L(GA)_nL}$, $\mathbf{L(GA)_nGL}$, wherein
n is an integer from 2 to 8;
each individual **A** segment is an amino acid sequence of from 8 to 18 amino acid residues, wherein from 0 to 3 of the amino acid residues are not Ala, and the remaining amino acid residues are Ala;
each individual **G** segment is an amino acid sequence of from 12 to 30 amino acid residues, wherein at least 40% of the amino acid residues are Gly; and each individual **L** segment is a linker amino acid sequence of from 0 to 20 amino acid residues; and
**CT** is a protein fragment having from 70 to 120 amino acid residues, which fragment is a C-terminal fragment derived from a spider silk protein.

**13.** Use of a spider silk protein according to claim 12 for producing polymers of the spider silk protein.

**14.** An isolated polynucleic acid molecule comprising a nucleic acid sequence selected from the group consisting of SEQ ID NO: 14-16; nucleic acid sequences encoding SEQ ID NO: 3-5; nucleic acid sequences which encodes a spider silk protein according to claim 12; and their complementary nucleic acid sequences.

**15.** A method of producing a spider silk protein according to claim 12, comprising the steps of:

(i) expressing a polynucleic acid molecule which encodes said spider silk protein in a suitable host; and
(ii) isolating the protein obtained in step (i), optionally involving removal of lipopolysaccharides and other pyrogens.

```
Ea MaSp1   SHTTPWTNPGLAENFMNSFMQGLSSMPGFTASQLDDMSTIAQSMVQSIQSLAAQGRTSPNKLQALNMAFA
Lg MaSp1   QANTPWSSKANADAFINSFISSAQNTGSFSQDQMDDMSLIGNTLMTAMDNMG--GRITPSKLQALDMAFA
Lh MaSp1   QANTPWSSKANADAFINSFISAASNTGSFSQDQMEDMSLIGNTLMAAMDNMG--GRITPSKLQALDMAFA
Nc MaSp1   -QNTPWSSTELADAFINAFMNEAGRTGAFTADQLDDMSTIGDTIKTAMDKMARSNKSSKGKLQALNMAFA
At MaSp2   QGATPWENSQLAESFISRFLRFIGQSGAFSPNQLDDMSSIGDTLKTAIEKMAQSRKSSKSKLQALNMAFA
Lg MaSp2   ---LRWSSKDNADRFINAFLQAASNSGAFSSDQVDDMSVIGNTLMTAMDNMG--GRITPSKLQALDMAFA
Lh MaSp2   QANTPWSSKENADAFIGAFMNAASQSGAFSSDQIDDMSVISNTLMAAMDNMG--GRITQSKLQALDMAFA
Nim MaSp2  QANTPWSDTATADAFIQNFLGAVSGSGAFTPDQLDDMSTVGDTIMSAMDKMARSNKSSKSKLQALNMAFA
Nc MaSp2   QARSPWSDTATADAFIQNFLAAVSGSGAFTSDQLDDMSTIGDTIMSAMDKMARSNKSSQHKLQALNMAFA
Ab CySp1   AVPSVFSSPNLASGFLQCLTFGIGNSPAFPTQEQQDLDAIAQVILNAVSSNTGATASAR--AQALSTALA
Ncl CySp1  PVPSVFSSPSLASGFLGCLTTGIGLSPAFPFQEQQDLDDLAKVILSAVTSNTDTSKSAR--AQALSTALA
Lh TuSp1   ASVNIFNSPNAATSFLNCLRSNIESSPAFPFQEQADLDSIAEVILSDVSS-VNTASSAT--SLALSTALA
Nc flag    IANSPFSNPNTAEAFARSFVSNIVSSGEFGAQGAEDFDDIIQSLIQAQ-SMGKGRHDTKAKAKAMQVALA
Nlm flag   IVNSPFSNPNTAEAFARSFVSNVVSSGEFGAQGAEDFDDIIQSLIQAQ-SMGKGRHDTKAKAKAMQVALA

Ea MaSp1   SSMAEIAASEEGGGSLSTKTSSIASAMSNAFLQTTGVVNQPFINEITQLVSMFAQAGMNDV
Lg MaSp1   SSVAEIAASEG--GDLGVTTNAIADALTSAFYQTTGVVNNRFISEIRSLISMFAQASANDV
Lh MaSp1   SSVAEIAASEG--GDLGVTTNAIADALTSAFYQTTGVVNSRFISEIRSLIGMFAQASANDV
Nc MaSp1   SSMAEIAAVEQGGLSVDAKTNAIADSLNSAFYQTTGAANPQFVNEIRSLINMFAQSSANEV
At MaSp2   SSMAEIAVAEQGGLSLEAKTNAIASALSAAFLETTGYVNQQFVNEIKTLIFMIAQASSNEI
Lg MaSp2   SSVAEIAVADG--QNVGGATNAISNALRSAFYQTTGVVNNQFISEISNLINMFAQVSANEV
Lh MaSp2   SSVAEIAVADG--QNVGAATNAISDALRSAFYQTTGVVNNQFITGISSLIGMFAQVSGNEV
Nim MaSp2  SSMAEIAAVEQGGQSMDVKTNAIANALDSAFYMTTGSTNQQFVNEMRSLINMLSAAAVNEV
Nc MaSp2   SSMAEIAAVEQGGMSMAVKTNAIVDGLNSAFYMTTGAANPQFVNEMRSLISMISAASANEV
Ab CySp1   SSLTDLLIAESAESNYSNQLSELTGILSDCFIQTTGSDNPAFVSRIQSLISVLSQNADTNI
Ncl CySp1  SSLADLLISESSGSSYQTQISALTNILSDCFVTTTGSNNPAFVSRVQTLIGVLSQSSSNAI
Lh TuSp1   SSLAELLVTESAEEDIDNQVVALSTILSQCFVETTGSPNPAFVASVKSLLGVLSQSASNYE
Nc flag    SSIAELVIAESSGGDVQRKTNVISNALRNALMSTTGSPNEEFVHEVQDLIQMLSQEQINEV
Nlm flag   SSIAELVIAESSGGDVQRKTNVISNALRNALMSTTGSPNEEFVHEVQDLIQMLSQEQINEV
```

Fig 1

```
CThyb_Esp      SRLSSPEASS RVSSAVSNLV SSG-PTNSAA LSSTISNVVS QIGASNPGLS GCDVLVQALL EVVSALIHIL GSSSIGQVNY GSAGQATQLV GQSVYQALGE F
CTnat_Eau      SRLSSPSAVS RVSSAVSSLV SNG-QVNMAA LPNIISNISS SVSASAPGAS GCEVIVQALL EVITALVQIV SSSSVGYINP SAVNQITNVV ANAMAQVMG- -
AF350266_At1   SRLSSPGAAS RVSSAVTSLV SSGGPTNSAA LSNTISNVVS QISSSNPGLS GCDVLVQALL EIVSALVHIL GSANIGQVNS SGVGRSASIV GQSINQAFS- -
AY666062_Cm1   SHLSSPEASS RVSSAVSNLV SSG-STNSAA LPNTISNVVS QISSSNPGLS GCDVLVQALL EVVSALIHIL GSSSIGQVNY GSAGQATQIV ---------- -
AF350273_Lg1   SALAAPATSA RISSHASTLL SNG-PTNPAS ISNVISNAVS QISSSNPGAS SCDVLVQALL ELVTALLTII GSSNVGNVNY DSSGQYAQVV SQSVQNAFV- -
AY953074_Lh1   SALSAPATSA RISSHASALL SSG-PTNPAS ISNVISNAVS QISSSNPGAS ACDVLVQALL ELVTALLTII GSSNIGSVNY DSSGQYAQVV TQSVQNVFG- -
AY666068_Mh1   SHLSSPEASS RVSSAVSNLV SGG-STNSAA LPNTISNVVS QISSSNPGLS GCDVLVQALL EVVSALIHIL GSSSIGQVDY GSAGQATQIV GQSA------ -
U20329_Nc1     SRLSSPQASS RVSSAVSNLV ASG-PTNSAA LSSTISNVVS QIGASNPGLS GCDVLIQALL EVVSALIQIL GSSSIGQVNY GSAGQATQIV GQSVYQALG- -
AY666076_Np1   SRLSSPEASS RVSSAVSNLV SSG-PTNSAA LSNTISNVVS QISSSNPGLS GCDVLVQALL EVVSALIHIL GSSSIGQVNY GSAGQATQIV ---------- -
AF350277_Nm1   SRLSSPQASS RVSSAVSNLV ASG-PTNSAA LSSTISNAVS QIGASNPGLS GCDVLIQALL EVVSALIHIL GSSSIGQVNY GSAGQATQ-- ---------- -
AF350279_Ns1   SRLSSPEASS RVSSAVSNLV SSG-PTNSAA LSSTISNVVS QIGASNPGLS GCDVLIQALL EVVSALVHIL GSSSIGQVNY GSAGQATQ-- ---------- -
AY666057_Ov1   SRLSSPEASS RVSSAVSNLV SSG-PTNSAA LSNTISNVVS QISSSNPGLS GCDVLVQALL EVVSAPIHIL GSSSIGQVNY GSAGQATQIV ---------- -
AY666064_Ps1   SRLSSPEASS RVSSAVSNLV SSG-PTNSAA LPNTISNVVS QISSSNPGLS GCDVLVQALL EVVSALIHIL GSSSIGQVNY GSAGQATQIV ---------- -
AF350285_Tk1   SLLSSPASNA RISSAVSALA SGA-ASGPGY LSSVISNVVS QVSSNSGGLV GCDTLVQALL EAAAALVHVL ASSSGGQVNL NTAGYTSQL- ---------- -
AF350286_Tv1   SRLSSPASNA RISSAVSALA SGG-ASSPGY LSSIISNVVS QVSSNNDGLS GCDTVVQALL EVAAALVHVL ASSNIGQVNL NTAGYTSQL- ---------- -
ABU20328_Ab2   SRLSSSAASS RVSSAVSSLV SSG-PTTPAA LSNTISSAVS QISASNPGLS GCDVLVQALL EVVSALVHIL GSSSVGQINY GASAQYAQMV ---------- -
AY365016_Aam2  -RLSSPQASS RVSSAVSTLV SSG-PTNPAS LSNAIGSVVS QVSASNPGLP SCDVLVQALL EIVSALVHIL GSSSIGQINY SASSQYARLV GQSIAQALG- -
AF350263_Aau2  SRLSSPQASS RVSSAVSTLV SSG-PTNPAA LSNAISSVVS QVSASNPGLS GCDVLVQALL ELVSALVHIL GSSSIGQINY AAS------- ---------- -
AF350267_At2   SRLSSPQASS RVSSAVSTLV SSG-PTNPAS LSNAISSVVS QVSSSNPGLS GCDVLVQALL EIVSALVHIL GSSSIGQINY AASSQYAQLV GQSLTQALG- -
AF350272_Gm2   SRLSSPQAGA RVSSAVSALV ASG-PTSPAA VSSAISNVAS QISASNPGLS GCDVLVQALL EIVSALVSIL SSASIGQINY GASGQYAAMI ---------- -
AF350275_Lg2   SALSSPTTHA RISSHASTLL SSG-PTNSAA ISNVISNAVS QVSASNPGSS SCDVLVQALL ELITALISIV DSSNIGQVNY GSSGQYAQMV G--------- -
AY953075_Lh2   SALSSPTTHA RISSHASTLL SSG-PTNAAA LSNVISNAVS QVSASNPGSS SCDVLVQALL EIITALISIL DSSSVGQVNY GSSGQYAQIV GQSMQQAMG- -
AY654293_Nc2   SRLASPDSGA RVASAVSNLV SSG-PTSSAA LSSVISNAVS QIGASNPGLS GCDVLIQALL EIVSACVTIL SSSSIGQVNY GAASQFAQVV GQSVLSAF-- -
AF350278_Nm2   SRLASPDSGA RVASAVSNLV SSG-PTSSAA LSSVISNAVS QIGASNPGLS GCDVLIQALL EIVSACVTIL SSSSIGQVNY GAA------- ---------- -
AF350280_Ns2   SRLASPDSGA RVASAVSNLV SSG-PTSSAA LSSVIXNAVS QIGASNPGLS GCDVLIXALL EIVSACVTIL SSSSIGQVNY GAA------- ---------- -
AF350269_DtFb1 SRLSSPEAAS RVSSAVSSLV SNG-QVNVDA LPSIISNLSS SISASATTAS DCEVLVQVLL EVVSALVQIV CS-------- ---------- ---------- -
AF350270_DtFb2 SRLSSPQAAS RVSSAVSSLV SNG-QVNVAA LPSIISSLSS SISASSTAAS DCEVLVQVLL EIVSALVQIV SSANVGYINP EASGSLN-AV GSALAAAMG- -
U47853_ADF1    NRLSSAGAAS RVSSNVAAIA SAG----AAA LPNVISNIYS GVLSS--GVS SSEALIQALL EVISALIHVL GSASIGNVSS VGVNSALNAV QNAVGAYAG- -
U47854_ADF2    SRLSSPSAAA RVSSAVS-LV SNGGPTSPAA LSSSISNVVS QISASNPGLS GCDILVQALL EIISALVHIL GSANIGPVNS SSAGQSASIV GQSVYRALS- -
U47855_ADF3    SRLSSPAASS RVSSAVSSLV SSG-PTKHAA LSNTISSVVS QVSASNPGLS GCDVLVQALL EVVSALVSIL GSSSIGQINY GASAQYTQMV GQSVAQALA- -
U47856_ADF4    SVYLRLQPRL EVSSAVSSLV SSG-PTNGAA VSGALNSLVS QISASNPGLS GCDALVQALL ELVSALVAIL SSASIGQVNV SSVSQSTQMI SQALS----- -

Consensus      SRLSSPQASS RVSSAVSNLV SSG-PTNSAA LSNTISNVVS QISASNPGLS GCDVLVQALL EVVSALVHIL GSSSIGQVNY GSAGQATQIV GQSVAQALGE F
```

Fig 2

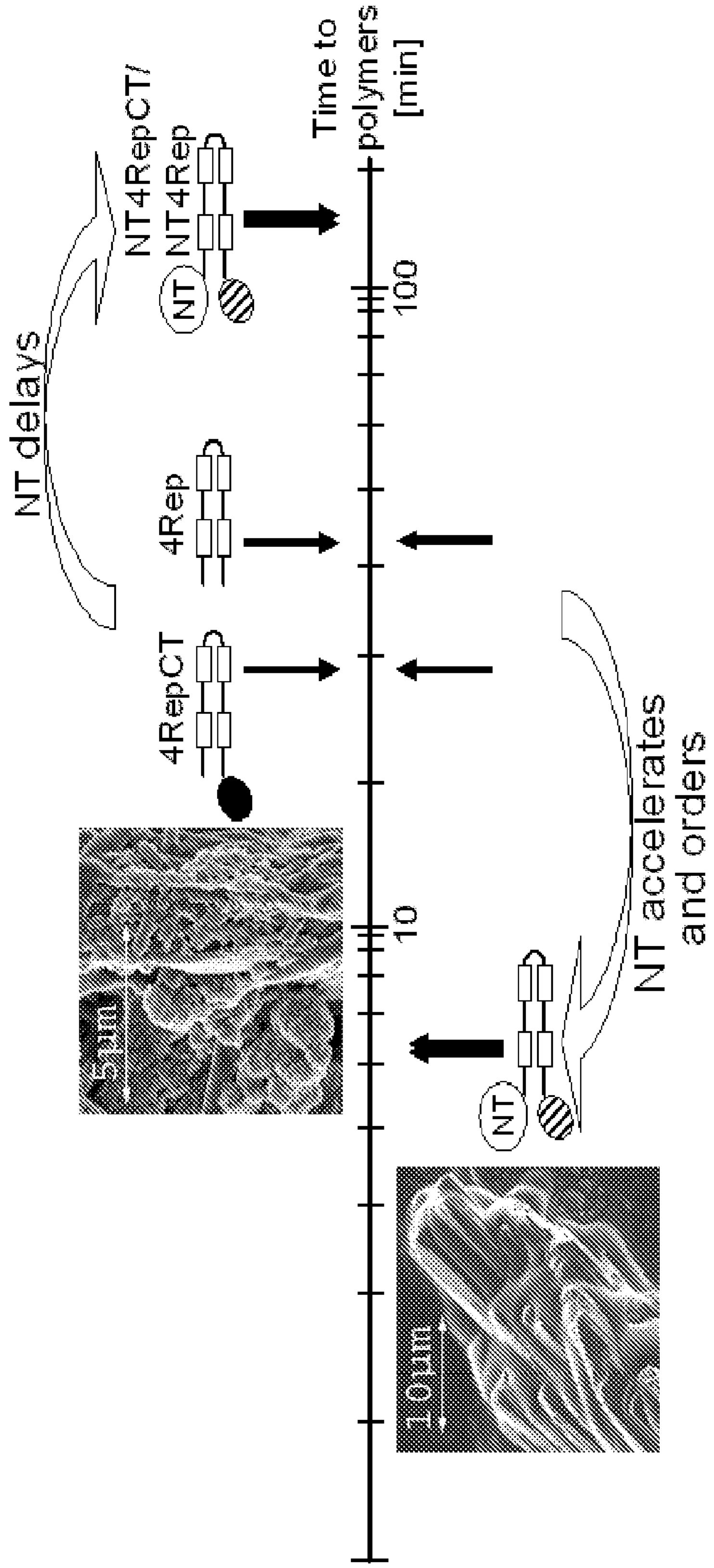
NT delays
4RepCT
4Rep
NT4RepCT/
NT4Rep
NT
Time to
polymers
[min]
100
10
NT accelerates
and orders
5µm
10µm

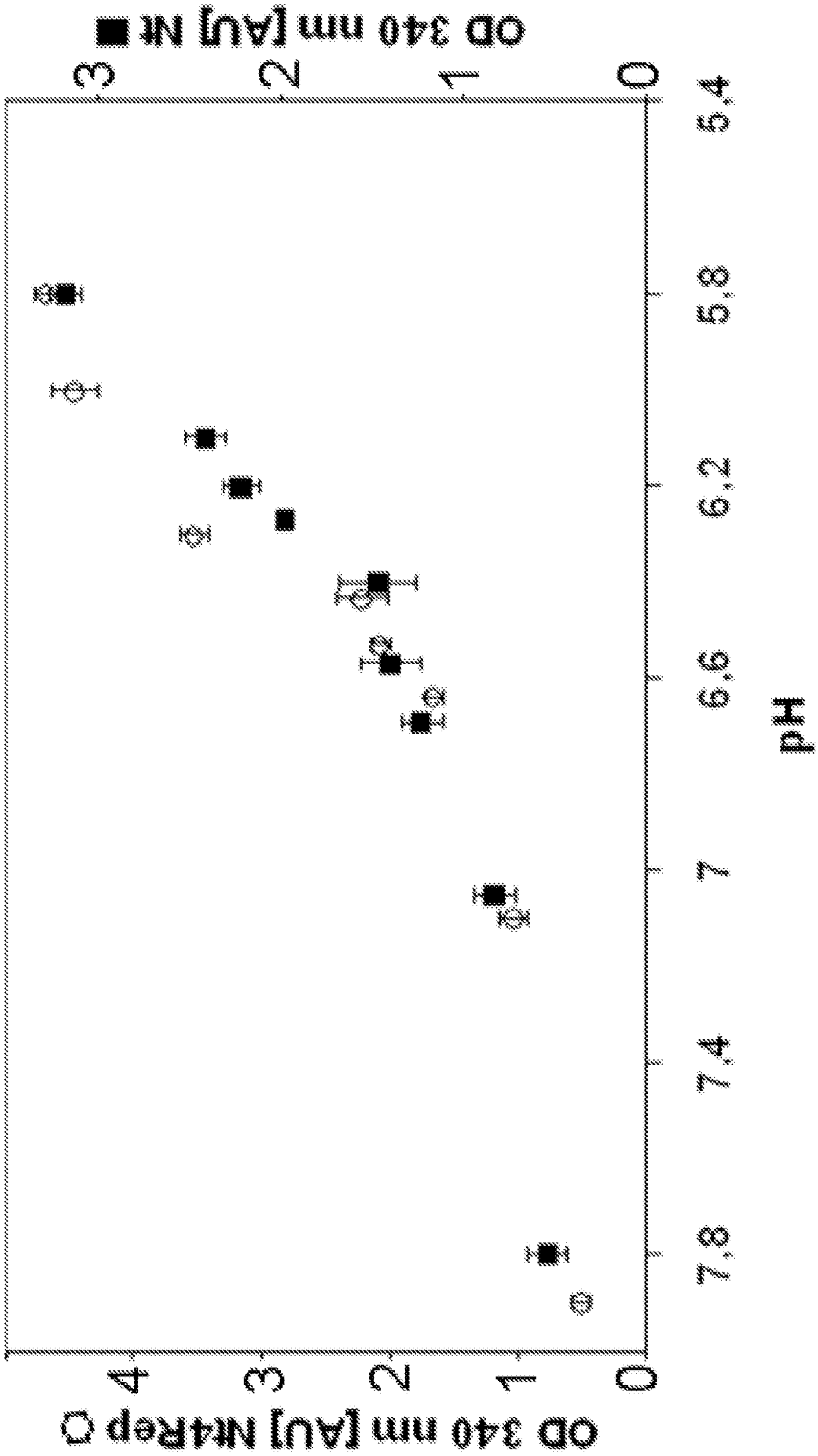


Fig 4

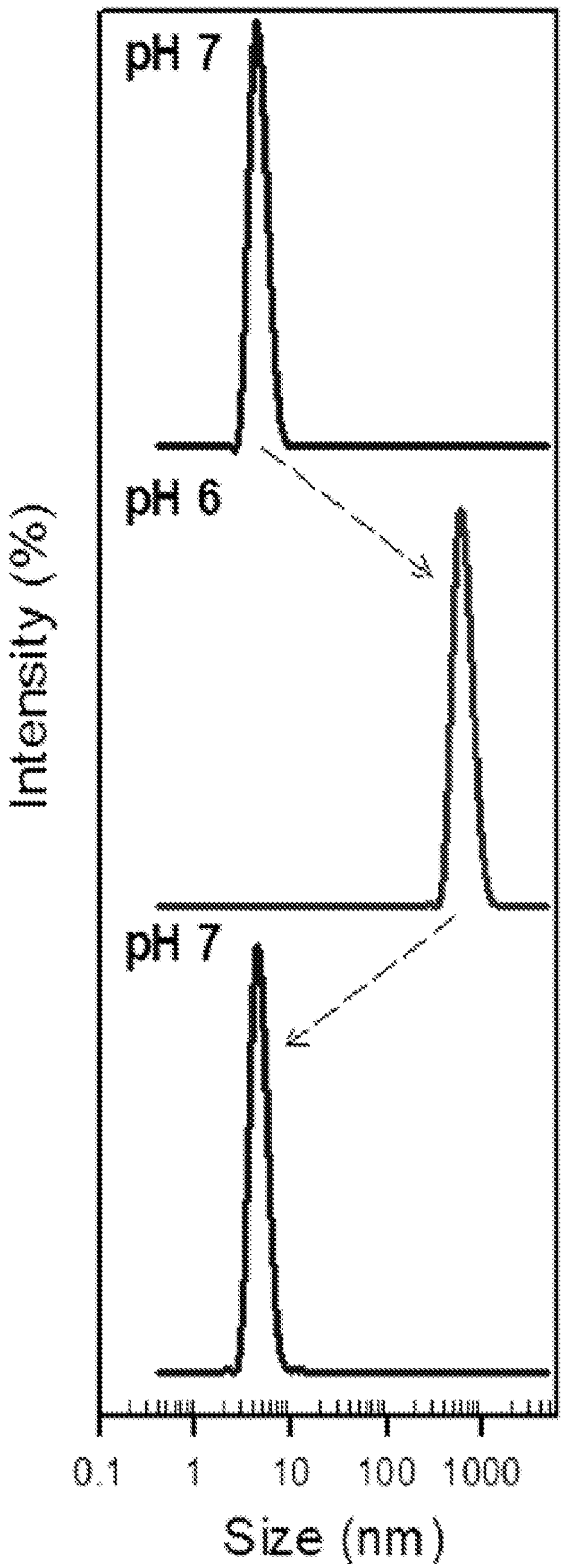


Fig 5

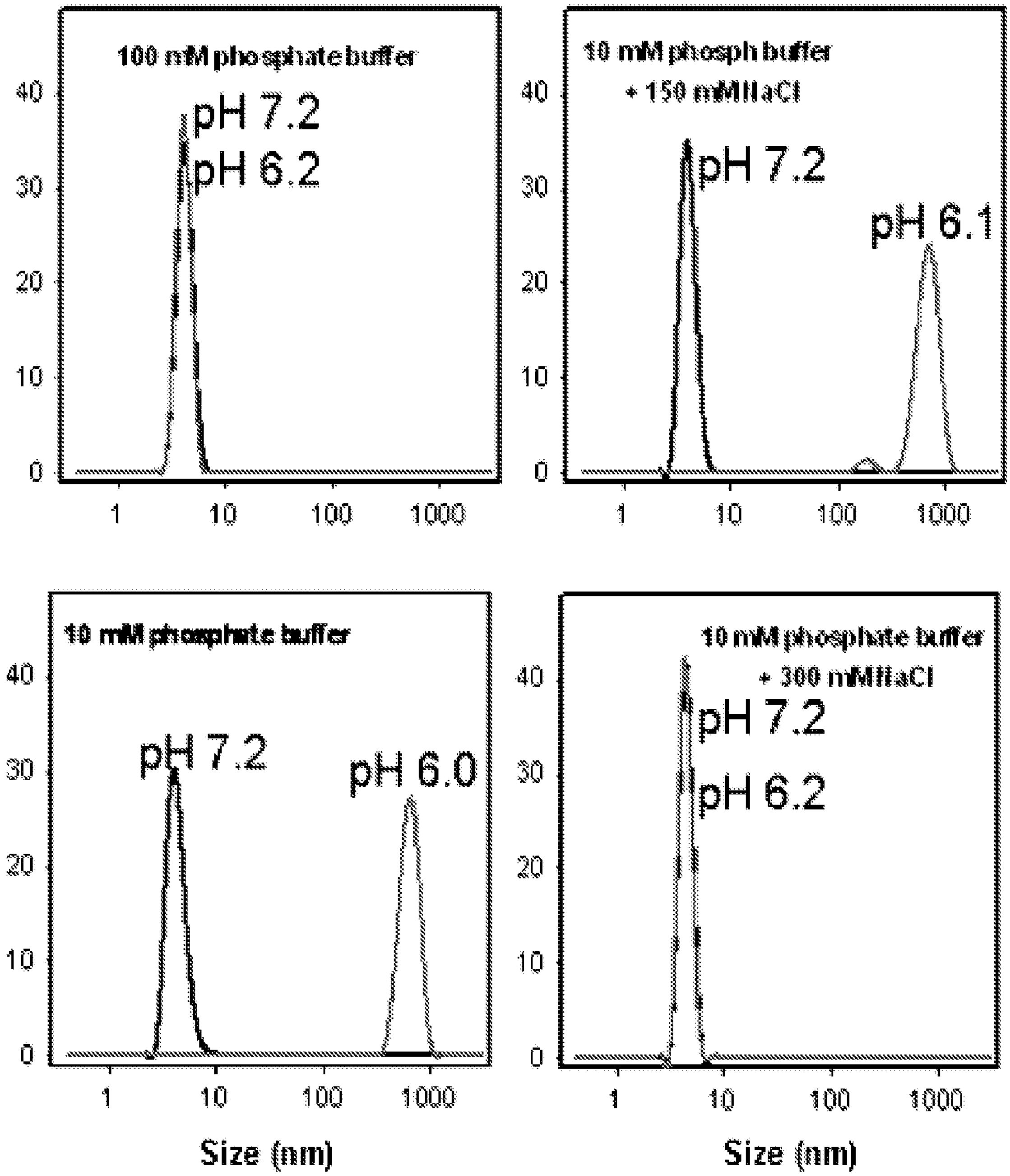
100 mM phosphate buffer
pH 7.2
pH 6.2
10 mM phosph buffer
+ 150 mM NaCl
pH 7.2
pH 6.1
10 mM phosphate buffer
pH 7.2
pH 6.0
10 mM phosphate buffer
+ 300 mM NaCl
pH 7.2
pH 6.2
0
10
20
30
40
1
10
100
1000
Size (nm)

Fig 6

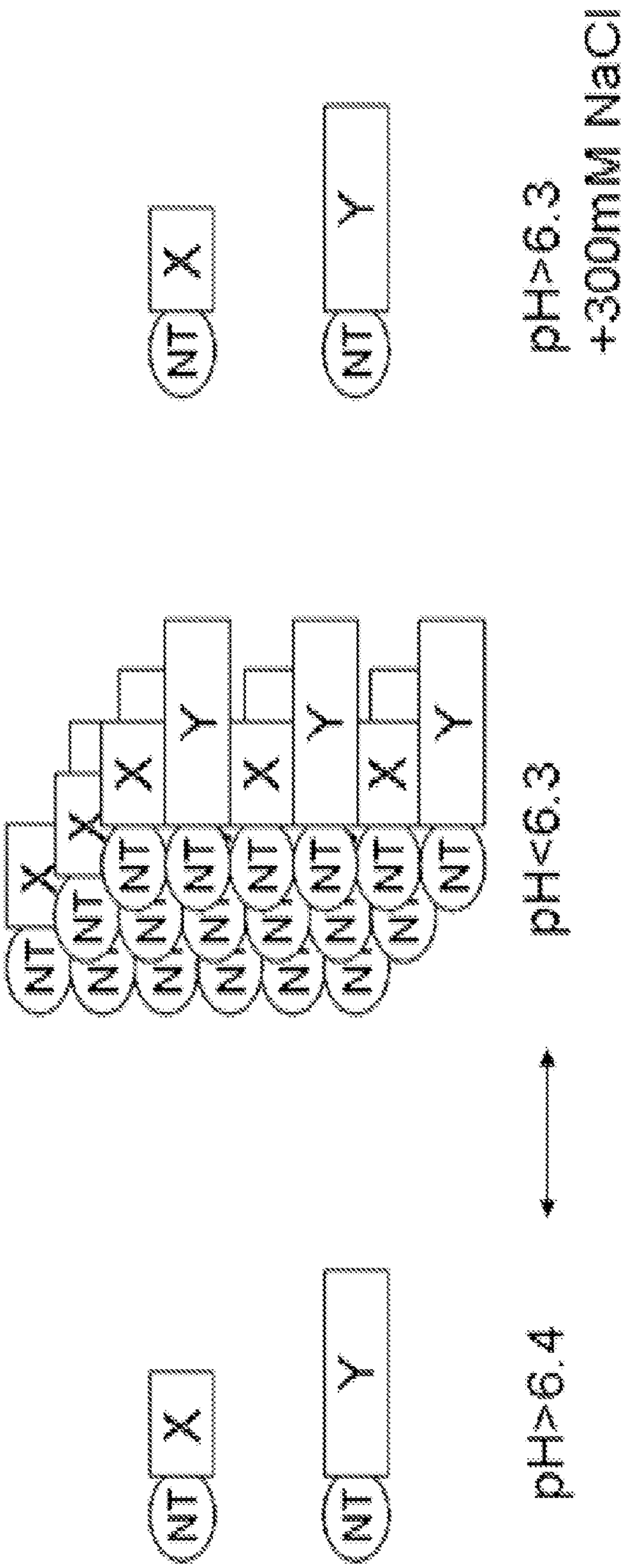


Fig 7

Europäisches Patentamt
European Patent Office
Office européen des brevets

# EUROPEAN SEARCH REPORT

Application Number
EP 09 15 8445

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/057727 A (NEXIA BIOTECH INC [CA]; KARATZAS COSTAS N [CA]; TURCOTTE CARL [CA]) 17 July 2003 (2003-07-17) * the whole document * ----- | 8-13,15 | INV. C07K14/435 D01F4/00 C12N15/62 |
| X | LEWIS RANDOLPH V ET AL: "Expression and purification of a spider silk protein: A new strategy for producing repetitive proteins" PROTEIN EXPRESSION AND PURIFICATION, ACADEMIC PRESS, SAN DIEGO, CA, vol. 7, no. 4, 1 January 1996 (1996-01-01), pages 400-406, XP002367349 ISSN: 1046-5928 * the whole document * ----- | 8-13,15 | |
| X | DATABASE EMBL [Online] 6 October 2006 (2006-10-06), "Euprosthenops australis partial mRNA for major ampullate spidroin 1 (MaSp1 gene)" XP002547537 retrieved from EBI accession no. EMBL:AM259067 Database accession no. AM259067 * the whole document * ----- | 12,14 | TECHNICAL FIELDS SEARCHED (IPC) C07K |
| X | DATABASE UniProt [Online] 14 November 2006 (2006-11-14), "SubName: Full=Major ampullate spidroin 1; Flags: Precursor; Fragment;" XP002547538 retrieved from EBI Database accession no. Q05H60_9ARAC * the whole document * ----- -/-- | 12,14 | |

The present search report has been drawn up for all claims

1

| Place of search | Date of completion of the search | Examiner |
|---|---|---|
| Munich | 25 September 2009 | Ury, Alain |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 15 8445

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HEDHAMMAR MY ET AL: "Structural properties of recombinant nonrepetitive and repetitive parts of major ampullate spidroin 1 from Euprosthenops australis: Implications for fiber formation" BIOCHEMISTRY, vol. 47, no. 11, March 2008 (2008-03), pages 3407-3417, XP002547535 ISSN: 0006-2960 | 1-7 | |
| Y | * the whole document * | 1-7 | |
| Y | DATABASE MEDLINE [Online] US NATIONAL LIBRARY OF MEDICINE (NLM), BETHESDA, MD, US; May 2007 (2007-05), PAN HONG-CHUN ET AL: "[Cloning and prokaryotic expression of major ampullate spidroin gene of spider]" XP002547539 Database accession no. NLM17577991 * abstract * & SHENG WU GONG CHENG XUE BAO = CHINESE JOURNAL OF BIOTECHNOLOGY MAY 2007, vol. 23, no. 3, May 2007 (2007-05), pages 446-451, ISSN: 1000-3061 | 1-7 | TECHNICAL FIELDS SEARCHED (IPC) |
| A | STARK MARGARETA ET AL: "Macroscopic fibers self-assembled from recombinant miniature spider silk proteins" BIOMACROMOLECULES, ACS, WASHINGTON, DC, US, vol. 8, no. 5, 1 May 2007 (2007-05-01), pages 1695-1701, XP002453664 ISSN: 1525-7797 | | |
| A | WO 2007/078239 A (JOHANSSON JAN [SE]; HJAELM GOERAN [SE]; STARK MARGARETA [SE]; RISING A) 12 July 2007 (2007-07-12) | | |
| | -/-- | | |

The present search report has been drawn up for all claims

1

| Place of search | Date of completion of the search | Examiner |
|---|---|---|
| Munich | 25 September 2009 | Ury, Alain |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 15 8445

| Category | Citation of document with indication, where appropriate, of relevant passages | Relevant to claim | CLASSIFICATION OF THE APPLICATION (IPC) |
|---|---|---|---|
| A | GAINES W A IV ET AL: "Identification and characterization of multiple Spidroin 1 genes encoding major ampullate silk proteins in Nephila clavipes" INSECT MOLECULAR BIOLOGY, vol. 17, no. 5, October 2008 (2008-10), pages 465-474, XP002547536 ISSN: 0962-1075<br>----- | | |
| A | MOTRIUK-SMITH DAGMARA ET AL: "Analysis of the conserved N-terminal domains in major ampullate spider silk proteins" BIOMACROMOLECULES, ACS, WASHINGTON, DC, US, vol. 6, no. 6, 10 August 2005 (2005-08-10), pages 3152-3159, XP002453721 ISSN: 1525-7797<br>----- | | |
| | | | TECHNICAL FIELDS SEARCHED (IPC) |

The present search report has been drawn up for all claims

1

| Place of search | Date of completion of the search | Examiner |
|---|---|---|
| Munich | 25 September 2009 | Ury, Alain |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 09 15 8445

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2009

| Patent document cited in search report | Publication date | Patent family member(s) | Publication date |
|---|---|---|---|
| WO 03057727 A | 17-07-2003 | AU 2003201513 A1 | 24-07-2003 |
| | | US 2007260039 A1 | 08-11-2007 |
| WO 2007078239 A | 12-07-2007 | AU 2006333611 A1 | 12-07-2007 |
| | | CA 2635660 A1 | 12-07-2007 |
| | | CN 101395178 A | 25-03-2009 |
| | | EP 1976868 A2 | 08-10-2008 |
| | | JP 2009521921 T | 11-06-2009 |
| | | US 2009226969 A1 | 10-09-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03057727 A
- WO 07078239 A
- WO 2007078239 A


### Non-patent literature cited in the description


- **Ayoub NA et al.** *PLoS ONE,* 2007, vol. 2 (6), 514
- **Huemmerich, D. et al.** *Curr. Biol.,* 2004, vol. 14, 2070-2074
- **Rising, A. et al.** *Biomacromolecules,* 2006, vol. 7, 3120-3124
- **Huemmerich, D. et al.** *Biochemistry,* 2004, vol. 43, 13604-13612
- **Lazaris, A. et al.** *Science,* 2002, vol. 295, 472-476
- **Lewis, R.V. et al.** *Protein Expr. Purif.,* 1996, vol. 7, 400-406
- **Fahnestock, S. R. ; Irwin, S. L.** *Appl. Microbiol. Biotechnol.,* 1997, vol. 47, 23-32
- **Arcidiacono, S. et al.** *Appl. Microbiol. Biotechnol.,* 1998, vol. 49, 31-38
- **Fahnestock, S. R. ; Bedzyk, L. A.** *Appl. Microbiol. Biotechnol.,* 1997, vol. 47, 33-39
- **Stark, M. et al.** *Biomacromolecules,* 2007, vol. 8, 1695-1701
- **Hedhammar, M. et al.** *Biochemistry,* 2008, vol. 47, 3407-3417
- **Rising A. et al.** *Biomacromolecules,* 2006, vol. 7, 3120-3124
- **Thompson, J.D. ; Higgins, D.G ; Gibson, T.J.** *Nucleic Acids Research,* 1994, vol. 22, 4673-4680